# EUROPEAN PATENT APPLICATION

(11) **EP 4 830 786 A2**
(43) Date of publication of application: **28.10.2026**
(21) Application number: 26198043.7
(22) Date of filing: 14.05.2021
(51) Int. Cl.: A61P 31/14

(54) **TLR7/8 ANTAGONISTS FOR USE IN THE TREATMENT OF CORONAVIRUS INFECTIONS**

(30) Priority: 14.05.2020 US 202063024683 P; 18.06.2020 US 202063040643 P
(62) Document number(s) of the earlier application(s) in accordance with Art. 76 EPC: 21731629.8 / 4 149 463
(71) Applicant: Merck Healthcare KGaA, 64293 Darmstadt (DE)
(72) Inventor: DEMARTINO, Julie A., Billerica, 01821 (US)
(74) Representative: Merck Serono S.A. Intellectual Property

(57) **Abstract**

The present invention encompasses TLR7/8 antagonists for use in the treatment of coronavirus infections, including COVID-19, alone or in combination with one or more additional therapeutic agents.

## Description

### TECHNICAL FIELD OF THE INVENTION

The present invention provides for toll-like receptor 7/8 (TLR7/8) inhibitors and their use in the treatment of coronavirus infections, including SARS-CoV infections such as COVID-19.

### BACKGROUND OF THE INVENTION

Toll-like receptors (TLR) currently comprising a gene family of 10 receptors with different specificities are part of the cellular pathogen pattern recognition system, which has evolved for defense against a variety of infections (bacteria, virus, fungi). Activation of TLRs leads to cytokine responses, e.g. with release of interferons and activation of specified immune cells. The functional expression of selected TLRs in tissues is highly different. Part of the receptors are located at the cell surface such as TLR4 (stimulated by E. coli lipopolysaccharide LPS), e.g. on epithelial cells, or TLR3, 7, 8 and 9 located at endosomal membranes in specified immune cells. The latter are all activated by nucleic acids, but recognize various types of them. For instance, TLR9 is activated by single stranded DNA containing CpG subsequences, TLR7 and 8 are activated by single stranded RNA, and TLR3 is activated by double-stranded RNA.

TLRs have been implicated in various autoimmune and inflammatory diseases, with the clearest example being the role played by TLR7 in the pathogenesis of systemic lupus erythematosus (Barrat and Coffman, Immunol Rev, 223:271-283, 2008). Additionally, a TLR8 polymorphism has been associated with rheumatoid arthritis (Enevold et al., J Rheumatol, 37:905-10, 2010).

### Coronaviruses

Coronaviruses (CoVs) are positive-sense, single-stranded RNA (ssRNA) viruses of the order *Nidovirales*, in the family *Coronaviridae*. There are four sub-types of coronaviruses - alpha, beta, gamma and delta - with the *Alphacoronaviruses* and *Betacoronaviruses* infecting mostly mammals, including humans. Over the last two decades, three significant novel coronaviruses have emerged which jumped from a non-human mammal hosts to infect humans: the severe acute respiratory syndrome (SARS-CoV-1) which appeared in 2002, Middle East respiratory syndrome (MERS-CoV) which appeared in 2012, and COVID-19 (SARS-CoV-2) which appeared in late 2019. In the first five months of identification of SARS-CoV-2, over 4 million people are known to have been infected, and almost 300,000 people have died. Both numbers likely represent a significant undercount of the devastation wrought by the disesase.

### COVID-19

SARS-CoV-2 closely resembles SARS-CoV-1, the causative agent of SARS epidemic of 2002-03 (Fung, et al, Annu. Rev. Microbiol. 2019. 73:529-57). Severe disease has been reported in approximately 15% of patients infected with SARS-CoV-2, of which one third progress to critical disease (e.g. respiratory failure, shock, or multiorgan dysfunction (Siddiqi, et al, J. Heart and Lung Trans. (2020), doi: https://doi.org/10.1016/j.healun.2020.03.012, Zhou, et al, Lancet 2020; 395: 1054-62. https://doi.org/10.1016/S0140-6736(20)30566-3). Fully understanding the mechanism of viral pathogenesis and immune responses triggered by SARS-CoV-2 would be extremely important in rational design of therapeutic interventions beyond antiviral treatments and supportive care. Much is still being discovered about the various ways that COVID-19 impacts the health of the people that develop it.

Extraordinary GU-rich single-strand RNA identified from SARS coronavirus contributes an excessive innate immune response. *In vitro* experiments, the result showed the representative SARS-CoV ssRNAs had powerful immunostimulatory activities to induce considerable level of pro-inflammatory cytokine TNF-a, IL-6 and IL-12 release via the TLR7 and TLR8. (Li, et al, Microbes and Infection (15:2, 88-95 (2013)). Point mutation bias in SARS-CoV-2 variants results in increased ability to stimulate inflammatory responses. The degree of increase of U in SARS-CoV-2 variants correlates with enhanced production of cytokines, such as TNF-α and IL-6, in cell lines when compared with stimulation by the ssRNA sequence of the isolated virus in Wuhan. Consistent with previous report, the cytokine production from THP-1 stimulated by ssRNA were dependent on TLR7. (Kosuge, et al., Scientific Reports (10:17766 (2020)).

Severe acute respiratory syndrome (SARS)-Corona Virus-2 (CoV-2), the etiologic agent for coronavirus disease 2019 (COVID-19), has caused a pandemic effecting over four million people worldwide with a case fatality rate of 2-4% as of May 2020. The virus has a high transmission rate, likely linked to high early viral loads and lack of pre-existing immunity (He, et. al, Nat Med 2020 https://doi.org/10.1038/s41591-020-0869-5). It causes severe disease especially in the elderly and in individuals with comorbidities. The global burden of COVID-19 is immense, and therapeutic approaches are increasingly necessary to tackle the disease. Intuitive anti-viral approaches including those developed for enveloped RNA viruses like HIV-1 (lopinavir plus ritonavir) and Ebola virus (remdesivir) have been implemented in testing as investigational drugs (Grein et al, NEJM 2020 https://do1.org/10.1056/NEJMo0a2007016; Cao,et al, NEJM 2020 DOI: 10.1056/NEJMoa2001282). But given that many patients with severe disease present with immunopathology, host-directed immunomodulatory approaches are also being considered, either in a staged approach or concomitantly with antivirals (Metha, et al, The Lancet 2020; 395(10229) DOI: https://doi.org/10.1016/S0140-6736(20)30628-0, Stebbing, et al, Lancet Infect Dis 2020. https://doi.org/10.1016/S1473-3099(20)30132-8).

While there are many therapies being considered for use in treatment of COVID-19, there are as yet no approved medications to treat the disease, and no vaccine available. To date, treatment typically consists only of the available clinical mainstays of symptomatic management, oxygen therapy, with mechanical ventilation for patients with respiratory failure. Thus, there is an urgent need for novel therapies to address the different stages of the SARS-CoV-2 infectious cycle (Siddiqi, et al.).

### BRIEF DESCRIPTION OF THE DRAWINGS

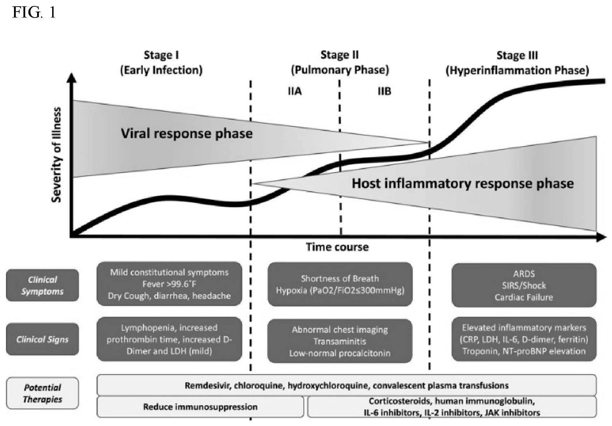

Figure 1 shows a schematic for disease progression which includes two phases: 1) viral response phase and 2) host inflammatory response phase. There are also three stages roughly identified with the disease, with the most severe cases being in Stage III where patients suffer from a severe cytokine storm.

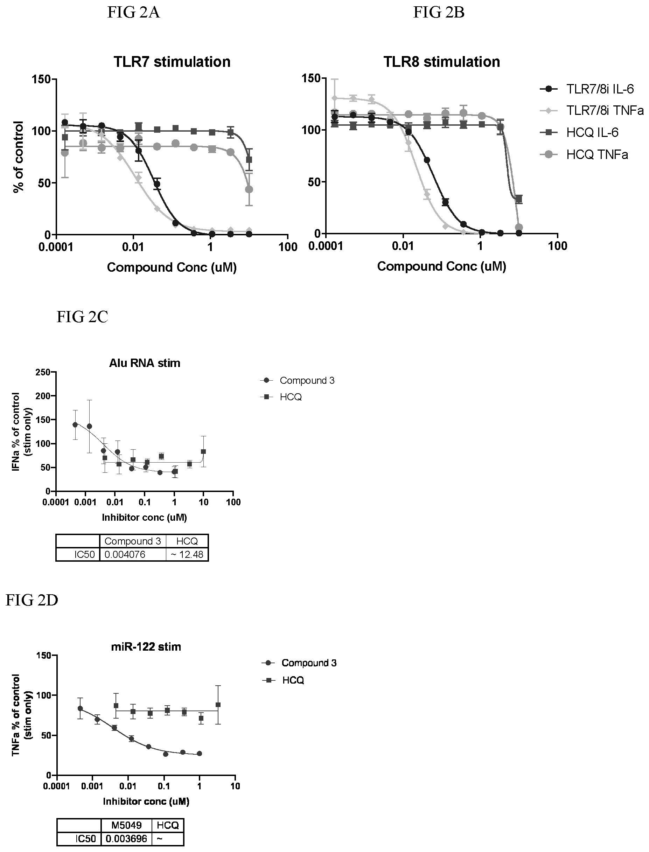

Figure 2A and 2B show the effect of Compound 3 vs. hydroxychloroquine administration on the production of IL-6 and TNF-alpha on samples stimulated with either TLR 7 or TLR 8 agonists. Compound 3 significantly reduces the amount of the cytokines seen as compared to hydroxychloroquine, even at low concentrations. Figures 2C shows the amount of IFN-alpha compared to control for cells with Alu stimulation when treated with Compound 3 or hydroxychloroquine, and Figure 2D shows the amount of TNF-alpha as compared to control for cells with miR-122 stimulation when treated with Compound 3 or hydroxychloroquine.

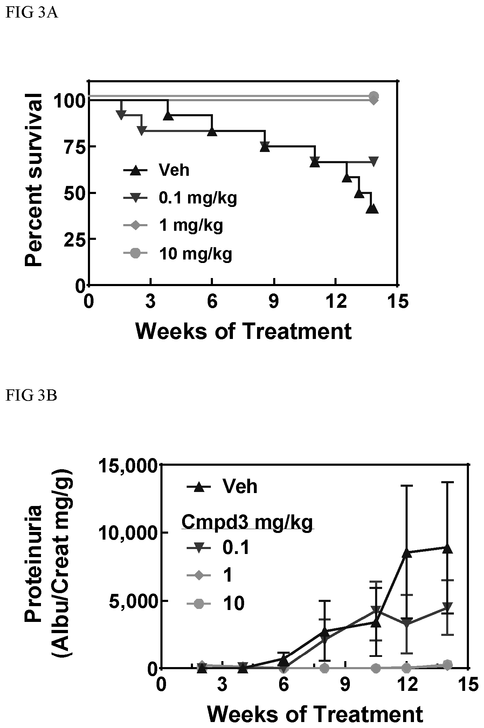

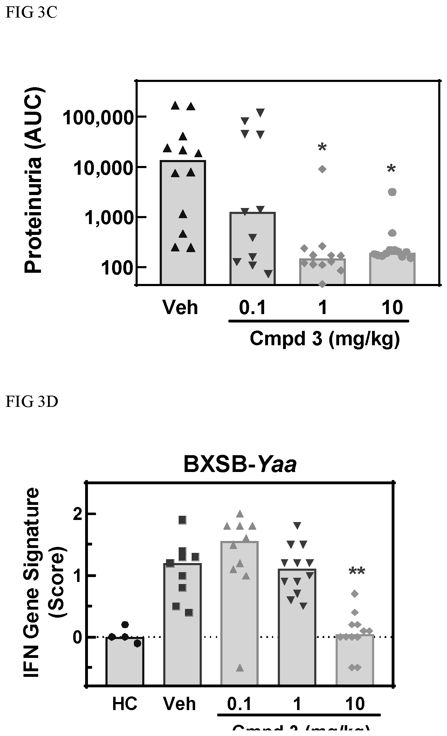

Figure 3A-3D show the results of administration of Compound 3 in mouse lupus models. Fig 3A shows survival over time (FIG 3A); Fig. 3B shows proteinuria as plotted as a timecourse; Fig 3C shows the AUC for individual mice for the timecourse; and FIG 3D shows blood gene expression analysis which was performed on a panel of 17 IFN-regulated genes to calculate an IFN gene signature score relative to a healthy control mice.

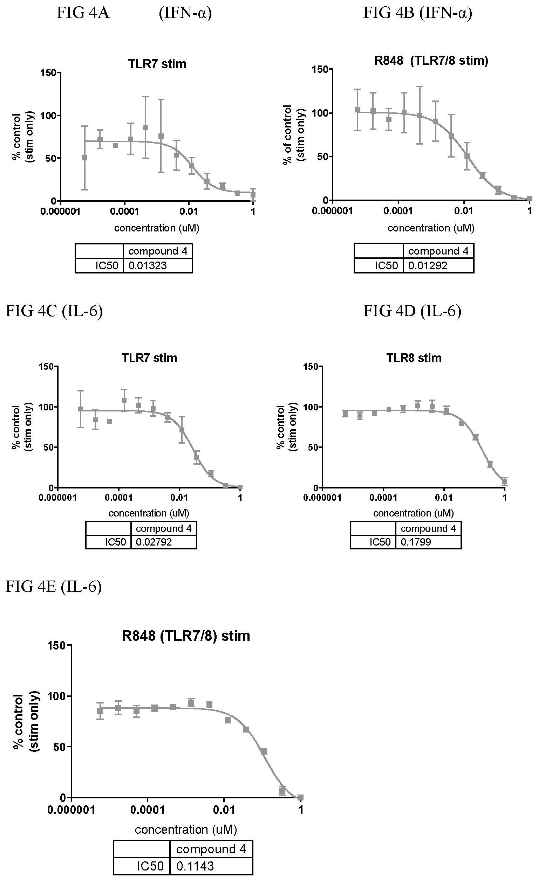

Figure 4A-4E show the effects of different concentrations of Compound 4 on plasma stimulated with different TLR 7, TLR 8, and TLR 7/8 agonists on the levels of IL-6 (FIG 4C-4E) and IFN-alpha (FIG. 4A, 4B).

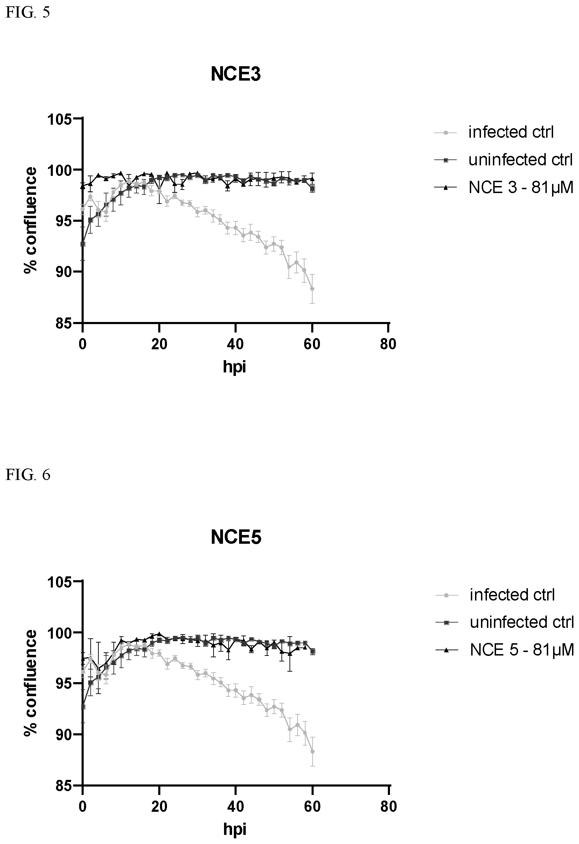

Figure 5 shows a schematic depicting the confluence of Calu-3 cells when treated with 81 µM of compound 4 ("NCE3") of the invention as compared to uninfected cells and infected cells without exposure to the therapeutic agent. The figure shows that confluence of the treated cells is similar to that of unifected cells in this experiment.
Figure 6 shows a schematic depicting the confluence of Calu-3 cells when treated with 81 µM of compound 3 ("NCE5") as compared to uninfected cells and infected control cells. The figure shows that confluence of the treated cells is similar to that of unifected cells in this experiment.

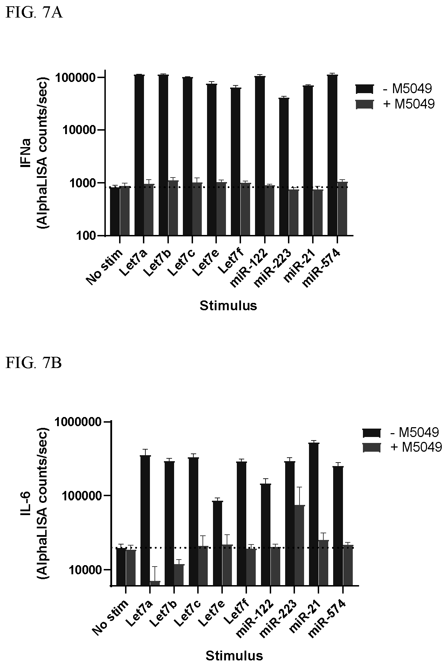

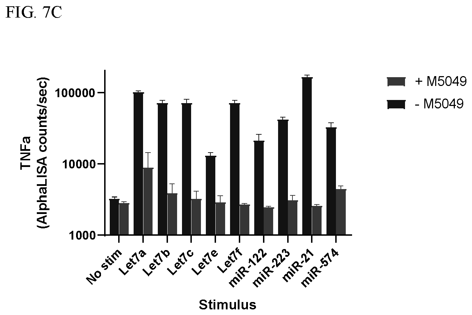

Figure 7A shows the concentration of IFN-alpha after stimulation in the presence of compound 3 (also described as "M5049") or in the absence of compound 3. Figure 7B shows the concentration of IL-6 after stimulation in the presence of compound 3 or the absence of compound 3. Figure 7C shows the concentration of TNF-alpha after stimulation in the presence or the absence of compound 3. All figures show the significant reduction of inflammatory cytokines when exposed to compound 3.

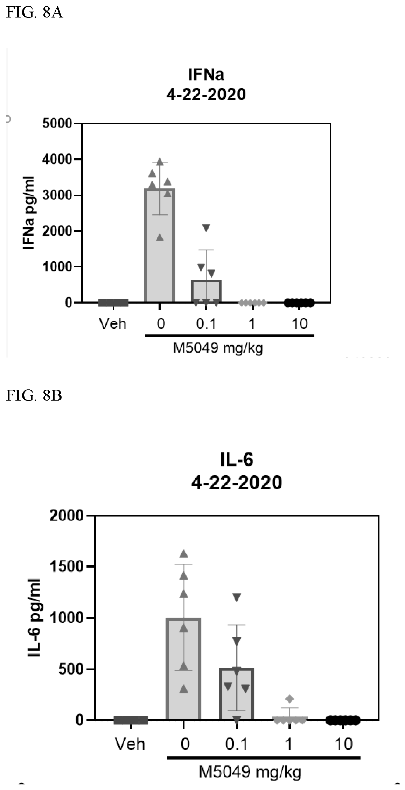

Figure 8A shows the reduction of IFN-alpha upon administration of compound 3 in an *in vivo* model. Figure 8B shows reduction of IL-6 upon administration of compound 3 in an *in vivo* model.

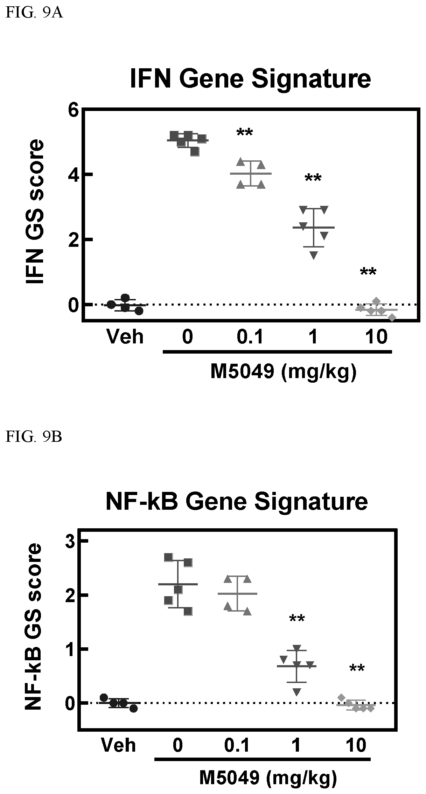

Figure 9A shows the IFN gene signature scores for various concentrations of compound 3 (M5049) - showing reduction of expression (correlating the reduction of inflammation in the lung) as dose of M5049 increases, with a complete reduction of IFN at 10 mg/kg of compound 3. Figure 9B shows NF-kB gene signature scores for various concentrations of compound 3 (M5049) - showing a reduction of expression (correlating to reduction of inflammation in the lung) of NF-kB as the dose of M5049 increases, with a complete reduction of NF-kB at 10 mg/kg.

### SUMMARY OF THE INVENTION

In one embodiment, the invention provides a TLR 7/8 inhibitor shown below: 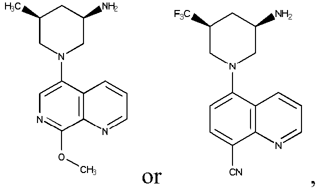 or a pharmaceutically acceptable salt thereof.

In another embodiment, the invention provides the TLR 7/8 inhibitors of the invention for use in the treatment of viral infections in a subject in need thereof. In one aspect of this embodiment, the viral infection is a coronavirus infection. In one aspect of this embodiment, the viral infection is a SARS-CoV-1, MERS-CoV, or SARS-CoV-2 infection. In one aspect of this embodiment, the viral infection is a SARS-CoV-2 infection.

One embodiment is a method of treating a coronavirus infection in a subject in need thereof, comprising administering an effective amount of a TLR 7/8 inhibitor, or a pharmaceutically acceptable salt thereof, to the subject. In one aspect of this embodiment, the subject is suffering from COVID-19 pneumonia. In another aspect, the subject is suffering from a hyperinflammatory host immune response due to a SARS-CoV-2 infection. In a further aspect of this embodiment, subject has a moderate to severe COVID-19 which requires medical intervention.

Another emobodiment of the present invention is a method of treating a coronavirus infection in a subject in need thereof comprising administering an effective amount of a TLR 7/8 inhibitor, or a pharmaceutically acceptable salt thereof, wherein the administration reduces the viral load in the subject. In one aspect of this embodiment, the TLR 7/8 inhibitor is administered prior to COVID-19 pneumonia development. In another aspect of this embodiment, the TLR 7/8 inhibitor is administered prior to the subject developing a severe cytokine storm. In a further aspect of this embodiment, the subject has a mild to moderate SARS-CoV-2 infection. In an additional aspect of this embodiment, the subject is asymptomatic at the start of the administration regimen.

### DETAILED DESCRIPTION

Given the dire need for therapies to address the powerful host inflammatory response phase of COVID-19, small molecule immunomodulatory compounds, such as the dual inhibitor of pattern recognition receptors TLR7 and TLR8 of the invention, may be a valuable tool in providing relief to COVID-19 patients. The compounds of the invention may inhibit a central mechanism of virus-associated cytokine storm in COVID-19 and may be targeted enough to stem severe immunopathology without compromising viral clearance.

At the initial antiviral response phase (Fig. 1), when the virus primarily infects ACE2-expressing specialized epithelial cells (type II pneumocytes) in the lung alveoli, direct anti-viral or immune-enhancing therapy (e.g. IFN-I, including Rebif) may prove to be of benefit in minimizing contagion and preventing progression to severe disease (Hoffmann, et al, Cell 2020. DOI: https://doi.org/10.1016/j.cell.2020.02.052; Sungnak, et al, Qbio preprint; arXiv:2003.06122 [q-bio.CB]; Zou, et al, Front Med 2020 https://doi.org/10.1007/s11684-020-0754-0; Zhao, et al, BioRxv preprint https://doi.org/10.1101/2020.01.26.919985; Qi, et al, BBRC 2020 https://doi.org/10.1016/j.bbrc.2020.03.044; Taccone, et al, Lancet Resp. Med. (2020) https://doi.org/10.1016/S2213-2600(20)30172-7). Indeed, recent papers have suggested a correlation between SARS-CoV-2 viral load, symptom severity and viral shedding (He, et al; Liu, et al, Lancet Infect Dis 2020. https://doi.org/10.1016/S1473-3099(20)30232-2). Antiviral drugs administered at symptom onset to blunt coronavirus replication are in the testing phase (Grein, et al; Taccone, et al).

In contrast, immunomodulatory treatments are proposed either in a staged approach to allow initial antiviral immune responses or concomitantly with antivirals in patients with progressive disease (Fig 1, stage II) (Stebbing, et al; Richardson, et al, Lancet 2020. https://doi.org/10.1016/S0140-6736(20)30304-4). Here, localized inflammation, systemic inflammatory markers, pulmonary disease and viral pneumonia are more evident, necessitating more supportive care (e.g. hospitalization, oxygen supplementation) (Siddiqi, et al). In this setting, anti-inflammatory therapy may be beneficial in preventing severe disease progression into a stage requiring mechanical ventilation.

Some current investigational immunomodulatory drugs are theorized to treat symptoms of cytokine storm associated with the host inflammatory phase of the illness (Fig. 1, Stage III). However, some medications currently being evaluated are too specific in their targeting to calm the cytokine storm (e.g., tocilizumab), too indiscrimate to be useful in calming the cytokine storm without causing too many adverse events (e.g., Jak1/2 inhibitors), are too weakly acting and/or non-specific in their targeting (e.g., hydroxychloroquine), and/or have serious side effects (Richardson, et al; Chen et al, medRxiv 2020 preprint doi: https://doi.org/10.1101/2020.03.22.20040758). However, none of the therapies currently being used in the clinic target the underlying driver to modulate the observed cytokine storm at its inception. Thus, even with mutliple medications being evaluated in clinical trials, there is still a dire need for an effective medication to calm the cytokine storm, and to reduce the viral load in patients suffering from COVID-19.

Stimulation of TLR7/8 activates an antiviral response (with IFN-I) and a proinflammatory cytokine response with production of IL-6 and TNF-alpha. The massive proinflammatory response (also called a "cytokine storm") after infection is the hallmark in severe cases of COVID-19 (Chow, et al, Annu. Rev. Immunol. 2018. 36:667-94; Huang, et al, Lancet (2020), 395:497-506). IL-6 is a cytokine featuring pleiotropic activity; it induces synthesis of acute phase proteins such as CRP, serum amyloid A, fibrinogen, and hepcidin in hepatocytes, among other effects. IL-6 also plays an important role on acquired immune response by stimulation of antibody production and of effector T-cell development. The primary role of another cytokine, TNF is in the regulation of immune cells; it is linked to inflammation and viral replication among many other effects. Thus, antagonizing TLR 7/8 should lead to a depression in both IL-6 and TNF-alpha, among other cytokines, and lead to a lessened proinflammatory response, and thus lessen or prevent the subject progressing to severe cytokine storm.

GU-containing RNA induces IFN-alpha, with the magnitude of the release correlated to the total number of GU per sequence. GU-rich sequences have originally been identified in HIV (Heil, et al, Science 2004 https://doi.org/10.1126/science.1093620) and are also found in human miRNA such as in the let-7 family and now in SARS-CoV-2 (Kosuge, et al., Scientific Reports (10:17766 (2020). Based on our internal analysis, it was found that the SARS-COV-2 genome has about 229 GU ssRNA fragments per sequence and alignment of a GU trimer sequence (GUUGUGUUGUGUUGU) containing 3 GUUGU motifs identified in HIV and known to be a strong activator of TLR7/8 identified 96 unique regions in the SARS-CoV-2 genome with at least 7 and up to 11 matching nucleotides. See Table 1:

| **SARS-CoV-2 sequence** | **ORF1 ab** | **S** | **ORF3 a** | **ORF** | **N** |
|---|---|---|---|---|---|
| GU-trimer matches (min 7 nt) | 53 | 5 | 2 | 1 | 1 |

The TLR7/8 inhibitors of the present invention inhibit GU-rich RNA-induced IFN-alpha and IL-6 secretion in experiments using GU=rich miRNA with sequences similar to the ones found in the SARS-CoV=2 genome. (See Figs. 7A-7C).

SARS-CoV-2 directly enters cells expressing ACE2 via receptor-mediated endocytosis (Hoffmann, et al). Successful viral replication requires host endosome acidification to release the viral genome into the host cytosol. Innate immune cells like monocytes, macrophages and neutrophils do not highly express ACE2, but have abundant Fc receptors (Zou, et al; Qi, et al; Lu, et al, Nat. Rev. Imm. 2018 https://doi.org/10.1038/nri.2017.106). In stage II (Fig.1), antibodies that bind the virus, can mediate viral uptake into myeloid cell endosomes via Fc receptors (FcR) or complement receptors (CR) (Lu, et al; Dandekar, et al, Nat. Rev. Imm. 2005, https://doi.org/10.1038/nri1732). Thus ACE2, FcR and CR present three mechanisms how SARS-CoV-2 can enter endosomes and trigger TLR7/8-driven hyperinflammation leading to cytokine storm and severe disease. Additionally, ssRNA virus can induce TLR7/8-dependent NETosis in neutrophils (Saitoh, et al, Cell Host Microbe (2012), 19;12(1):109-16) leading to release of DNA and RNA, creating a feed-forward loop to further fuel TLR7/8-driven inflammation (Herster et al, Nat Commun 2020; 11, 105 https://doi.org/10.1038/s41467-019-13756-4), which has been proposed as a driver of severe COVID 19 (Barnes, et al, J Exp med 2020; 217 (6) https://doi.org/10.1084/jem.20200652). SARS-CoV-1 derived ssRNA has been shown to mediate severe TLR7/8-driven lung pathology in animal models and presents as a potential driver of virus-associated cytokine storm (Li, et al, Microbes Infect 2013; 15 (2) 88-95. https://doi.org/10.1016/j.micinf.2012.10.008). Moreover, TLR8-expressing innate immune cell infiltrates have been observed in lungs of severely ill patients (Liao, et al, medRxiv preprint doi: https://doi.org/10.1101/2020.02.23.20026690). In this context, inhibiting TLR7/8 activation may be a unique targeted therapy that can quell the deleterious inflammatory cascade triggered by coronaviruses, including SARS-CoV-2, at the roots, thus providing selective immunosuppression yet uncompromised initial natural viral clearance.

Being able to slow the viral reproduction in the early stages of infection may allow the subject to avoid severe disease. This potential antiviral effect may be in addition to the dampening or avoidance of the cytokine storm associated with severe viral infections, as discussed above. Thus, the compounds of the inevention provide a unique opportunity to effectively treat subjects with coronavirus infections, such as SARS-CoV-2.

It is believed that compounds of the invention may change the pH at the surface of the cell membrane and, thus, inhibit the fusion of the virus to the cell membrane. It is hypothesized that compounds of the invention may inhibit nucleic acid replication, glycosylation of viral proteins, virus assembly, new virus particle transport, and/or virus release. The result of administration of a compound of the invention is to reduce viral replication, which in turn will reduce viral load, and reduce the severity of disease. Whatever the exact mechanism of action for the antiviral properties of the compounds of the invention, it is proposed that administration thereof may have one or more clinical benefits, as described further herein.

"COVID-19" is the name of the disease which is caused by a SARS-CoV-2 infection. While care was taken to describe both the infection and disease with accurate terminology, "COVID-19" and "SARS-CoV-2 infection" are meant to be roughly equivalent terms.

As of the writing of this application, the determination and characteristics of the severity of COVID-19 patients/symptoms has not been definitively established. However, in the context of this invention, "mild to moderate" COVID-19 occcurs when the subject presents as asymptomatic or with less severe clinical symptoms (e.g., low grade or no fever (<39.1°C), cough, mild to moderate discomfort) with no evidence of pneumonia, and generally does not require medical attention. When "moderate to severe" infection is referred to, generally patients present with more severe clinical symptoms (e.g., fever >39.1°C, shortness of breath, persistant cough, pneumonia, etc.). As used herein "moderate to severe" infection typically requires medical intervention, including hospitalization. During the progression of disease, a subject can transition from "mild to moderate" to "moderate to severe" and back again in one course of bout of infection.

Treatment of COVID-19 using the methods of this invention include administration of an effective amount of a TLR 7/8 inhibitor of the invention at any stage of the infection to prevent or reduce the symptoms associated therewith. Typically, subjects will be administered an effective amount of a TLR 7/8 inhibitor of the invention after definitive diagnosis and presentation with symptoms consistent with a SARS-CoV-2 infection, and administration will reduce the severity of the infection and/or prevent progression of the infection to a more severe state. The clinical benefits upon such administration is described in more detail in the sections below.

### 1. Compounds and Definitions

One embodiment is a compound selected from the group consisting of: 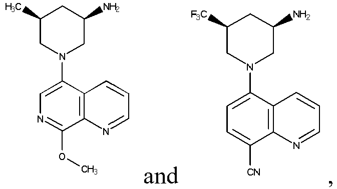 or a pharmaceutically acceptable salt thereof. In one aspect of this embodiment, the TLR 7/8 inhibitor of the invention is: 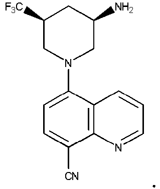 In another aspect of this embodiment, the TLR 7/8 inhibitor of the invention is:

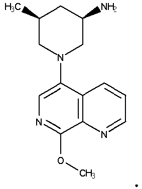

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention.

Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. In some embodiments, the group comprises one or more deuterium atoms.

### 2. Uses, Formulation and Administration

The term "patient" or "subject", as used herein, means an animal, preferably a human. However, "subject" can include companion animals such as dogs and cats. In one embodiment, the subject is an adult human patient. In another embodiment, the subject is a pediatric patient. Pediatric patients include any human which is under the age of 18 at the start of treatment. Adult patients include any human which is age 18 and above at the start of treatment. In one embodiment, the subject is a member of a high-risk group, such as being over 65 years of age, immunocompromised humans of any age, humans with chronic lung conditions (such as, asthma, COPD, cystic fibrosis, etc.), and humans with other co-morbidities. In one aspect of this embodiment, the other co-morbidity is obesity, diabetes, and/or hypertension.

Compositions of the present invention are administered orally, parenterally, by inhalation spray, topically, rectally, nasally, buccally, vaginally or via an implanted reservoir. Preferably, the compositions are administered orally. In one embodiment, the oral formulation of a compound of the invention is a tablet or capsule form. In another embodiment, the oral formulation is a solution or suspension which may be given to a subject in need thereof via mouth or nasogastric tube. Any oral formulations of the invention may be administered with or without food. In some embodiments, pharmaceutically acceptable compositions of this invention are administered without food. In other embodiments, pharmaceutically acceptable compositions of this invention are administered with food.

Pharmaceutically acceptable compositions of this invention are orally administered in any orally acceptable dosage form. Exemplary oral dosage forms are capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents are optionally also added.

The amount of compounds of the present invention that are optionally combined with the carrier materials to produce a composition in a single dosage form will vary depending upon the host treated, the particular mode of administration. Preferably, provided compositions should be formulated so that a dosage of between 0.01 - 100 mg/kg body weight/day of the compound can be administered to a patient receiving these compositions.

In one embodiment, the total amount of TLR 7/8 inhibitor administered to the subject in need thereof is between about 10 mg to about 500 mg per day. In one aspect of this embodiment, the total amount of TLR 7/8 inhibitor administered is between about 50 mg and about 300 mg per day. In another aspect, the total amount of TLR 7/8 inhibitor administered is between about 100 mg and about 200 mg per day.

In another embodiment, the TLR 7/8 inhibitor is administered once a day. In another aspect of this embodiment, the TLR 7/8 inhibitor is administered twice a day.

In one embodiment, the amount of TLR 7/8 inhibitor administered to the subject in need thereof is about 50 mg twice a day. In another embodiment, the amount of TLR 7/8 inhibitor administered to the subject in need thereof is about 100 mg twice a day.

In any of the above embodiments, the TLR 7/8 inhibitor is administered for a period of about 7 day to about 21 days. In one aspect of any of the above embodiments, the TLR 7/8 inhibitor is administered for about 14 days.

In one embodiment of the invention, 50 mg of the TLR 7/8 inhibitor of the invention is administered twice a day for about 14 days. In another embodiment of the invention, 100 mg of the TLR 7/8 inhibitor of the invention is administered twice a day for about 14 days.

In one embodiment of the invention, the subject is suffering from COVID-19 pneumonia. In one embodiment of the invention, the subject is suffering from an extreme proinflammatory response due to the COVID-19, which may present in any major organ of the body. In one embodiment of this invention, the subject is suffereing from acute respiratory distress syndrome (ARDS) due to COVID-19. In one embodiment of this invention, the subject is suffering from one or more symptoms selected from chest congestion, cough, blood oxygen saturation (SpO₂) levels below 94%, shortness of breath, difficulty breathing, fever, chills, repeated shaking with chills, muscle pain and/or weakness, headache, sore throat and/or new loss of taste or smell.

In one embodiment, the subject is suffering from a hyperinflammatory host immune response to a SARS-CoV-2 infection. In one aspect of this embodiment, the hyperinflammatory host immune response is associated with one or more clinical indications selected from 1) reduced levels of lymphocytes, especially natural killer (NK) cells in peripheral blood; 2) high levels of inflammatory parameters (eg, C reactive protein [CRP], ferritin, d-dimer), and pro-inflammatory cytokines (eg, IL-6, TNF-alpha, IL-8, and/or IL-1beta; 3) a deteriorating immune system demonstrated by lymphocytopenia and/or atrophy of the spleen and lymph nodes, along with reduced lymphocytes in lymphoid organs; 4) dysfunction of the lung physiology represented by lung lesions infiltrated with monocytes, macrophages, and/or neutrophils, but minimal lymphocytes infiltration resulting in decreased oxygenation of the blood; 5) acute respiratory distress syndrome (ARDS); 6) vasculitis; 7) encephalitis, Guillain-Barre syndrome, and other neurologic disorders; 8) kidney dysfunction and kidney failure; 9) hypercoagulability such as arterial thromboses; and 10) or any combination of above resulting in end-organ damage and death.

In one embodiment, the subject with COVID-19 is a pediatric patient suffering from vasculitis, including Kawasaki disease (i.e., Kawasaki syndrome) and Kawasaki-like disease.

In one embodiment of the invention, the subject is being treated inpatient in a hospital setting. In another embodiment, the subject is being treated in an outpatient setting. In one aspect of the preceding embodiments, the subject may continue administration of the TLR 7/8 inhibitor after being transitioned from being treated from an inpatient hospital setting to an outpatient setting.

In one embodiment, the administration of the TLR 7/8 inhibitor results in one or more clinical benefit. In one aspect of this embodiment, the one or more clinical benefit is selected from the group comprising: reduction of duration of a hospital stay, reduction of the duration of time in the Intensive Care Unit (ICU), reduction in the likelihood of the subject being admitted to an ICU, reduction in the rate of mortality, reduction in the likelihood of kidney failure requiring dialysis, reduction in the likelihood of being put on non-invasive or invasive mechanical ventilation, reduction of the time to recovery, reduction in the likelihood supplemental oxygen will be needed, improvement or normalization in the peripheral capillary oxygen saturation (SpO₂ levels) without mechanical intervention, reduction of severity of the pneumonia as determined by chest imaging (eg, CT or chest X ray), reduction in the cytokine production, reduction of the severity of acute respiratory distress syndrome (ARDS), reduction in the likelihood of developing ARDS, clinical resolution of the COVID-19 pneumonia, improvement of the PaO₂/FiO₂ ratio, and reduction of the inflammatory response in the subject.

In another embodiment, the one or more clinical benefits includes the improvement or normalization in the peripheral capillary oxygen saturation (SpO₂ levels) in the subject without mechanical ventilation or extracorporeal membrane oxygenation.

In one embodiment, the one or more clinical benefits includes the reduction of the inflammatory response of the subject. In one aspect of this embodiment, the reduction of the inflammatory response in the subject results in the reduction of proinflammatory cytokine release driven by NFκB (NF-kappa-B) IL-1b, IL-6, IL-8, IL-12, IL-18, IL-23, or IL-27, alone or in combination with inhibition of cytokine release driven by IRF3/7, such as type I IFNs, including IFN-alpha and/or IFN-beta. In one aspect of this embodiment, the one or more clinical benefits includes the avoidance of a severe cytokine storm in the subject.

In a further embodiment, the one of more clinical benefits is reduction in the likelihood of being hospitalized, reduction in the likelihood of ICU admission, reduction in the likelihood being intubated (invasive mechanical ventilation), reduction in the likelihood supplemental oxygen will be needed, reduction in the length of hospital stay, reduction in the likelihood of mortality, and/or a reduction in likelihood of relapse, including the likelihood of rehospitalization.

Methods of immunoregulation are provided by the present disclosure and include those that suppress and/or inhibit an immune response, including, but not limited to, an immune response. Immune suppression and/or inhibition according to the methods described herein may be practiced on individuals including those suffering from a disorder associated with an unwanted activation of an immune response, such as a viral infection.

The invention also provides a method of treating a viral infection in a subject in need thereof comprising administering an effective amount of a compound of the invention to the subject. An amount effective to treat or inhibit a viral infection is an amount that will cause a reduction in one or more of the manifestations of viral infection, such as viral lesions, viral load, rate of virus production, and mortality as compared to untreated control subjects.

Provided herein are methods of inhibiting an immune response in an individual, the method comprising administering to the individual at least one TLR inhibitor as disclosed herein in an amount effective to inhibit the immune response in the individual. In some variations, the immune response is associated with chronic pathogen stimulation. In some variations, the immune response is associated with viral infection. In further aspects, wherein inhibiting the immune response ameliorates one or more symptoms of the viral disease or disorder resulting from infection by SARS-CoV-2. In still further aspects, wherein inhibiting the immune response treats the viral disease or disorder resulting from infection by SARS-CoV-2. In yet further aspects, wherein inhibiting the immune response prevents or delays development of the viral disease or disorder resulting from infection by SARS-CoV-2. Other variations provided herein relate to immunoinhibitory therapy of individuals having been exposed to or infected with SARS-CoV-2. Administration of a TLR inhibitor to an individual having been exposed to or infected with SARS-CoV-2 results in suppression of COVID-19-induced excessive cytokine production. In some aspects, at least one TLR inhibitor is administered in an amount effective to suppress COVID-19 induced cytokine production in an individual exposed to or infected with SARS-CoV-2.

In one embodiment, the administration of the TLR 7/8 inhibitor selectively reduces the hyperinflammatory host immune response state while not interfering with the subject's natural interferon response to the viral infection. In one aspect of this embodiment, the hyperinflammatory host immune response state is reduced before the subject suffers a severe cytokine storm.

One embodiment of the invention is a method of treating a coronavirus infection in a subject in need thereof, comprising administering an effective amount of a TLR 7/8 inhibitor, or a pharmaceutically acceptable salt thereof, to the subject. In one aspect of this embodiment, the subject is infected with SARS-CoV-2. In another aspect of this embodiment, the administration of the TLR 7/8 inhibitor results in the reduction of the viral load in the subject. In a further aspect of this embodiment, administration of the TLR 7/8 inhibitor reduces the viral load by increasing the pH of the endosome, reducing the ability of the virus to enter cells, and/or interfering with the terminal glycosylation of cellular receptor ACE2. In another aspect of this embodiment, administration of the TLR 7/8 inhibitor of the invention provides reduction in viral replication. In a further aspect of this embodiment, administration of the TLR 7/8 inhibitor may inhibit one or more of nucleic acid replication, glycosylation of viral proteins, virus assembly, new virus particle transport, and virus release.

In one embodiment, the TLR 7/8 inhibitor is administered prior to COVID-19 pneumonia developing. In one embodiment, the TLR 7/8 inhibitor is administered prior to the subject developing a cytokine storm. In another embodiment, the subject has a mild to moderate SARS-CoV-2 infection. In a further embodiment, the subject is asymptomatic at the start of the administration regimen. In another embodiment, the subject has had known contact with a patient who has been diagnosed with a SARS-CoV-2 infection. In an additional embodiment, the subject begins administration of the TLR 7/8 inhibitor prior to being formally diagnosed with COVID-19.

One embodiment is a method of treating a subject with COVID-19 comprising administration of an effective amount of a TLR 7/8 inhibitor to the subject. In one aspect of this embodiment, the subject has been previously vaccinated with a SARS-CoV-2 vaccine and develops vaccine-related exacerbation of infection, for example, an antibody-dependent enhancement or related antibody-mediated mechanisms of vaccine/antibody-related exacerbation.

In any of the above embodiments, the administration of the TLR 7/8 inhibitor results in one or more clinical benefits to the subject. In one aspect of this embodiment, the one or more clinical benefits is shortening the duration of infection, reduction of the likelihood of hospitalization, reduction in the likelihood of mortality, reduction in the likelihood of ICU admission, reduction in the likelihood being placed on mechanical ventilation, reduction in the likelihood supplemental oxygen will be needed, and/or reduction in the length of hospital stay. In another aspect of this embodiment, the one or more clinical benefits is avoidance of a significant proinflammatory response. In a further aspect of this embodiment, the one or more clinical benefit is the failure of the subject to develop significant symptoms of COVID-19.

The compounds of the invention can be administered before or following an onset of SARS-CoV-2 infection, or after acute infection has been diagnosed in a subject. The aforementioned compounds and medical products of the inventive use are particularly used for the therapeutic treatment. A therapeutically relevant effect relieves to some extent one or more symptoms of a disorder, or returns to normality, either partially or completely, one or more physiological or biochemical parameters associated with or causative of a disease or pathological condition. Monitoring is considered as a kind of treatment provided that the compounds are administered in distinct intervals, e.g. in order to boost the response and eradicate the pathogens and/or symptoms of the disease. The methods of the invention can also be used to reduce the likelihood of developing a disorder or even prevent the initiation of disorders associated with COVID-19 in advance of the manifestation of mild to moderate disease, or to treat the arising and continuing symptoms of an acute infection.

Treatment of mild to moderate COVID-19 is typically done in an outpatient setting. Treatment of moderate to severe COVID-19 is typically done inpatient in a hospital setting. Additionally, treatment can continue in an outpatient setting after a subject has been discharged from the hospital.

The invention furthermore relates to a medicament comprising at least one compound according to the invention or a pharmaceutically salts thereof.

A "medicament" in the meaning of the invention is any agent in the field of medicine, which comprises one or more compounds of the invention or preparations thereof (e.g. a pharmaceutical composition or pharmaceutical formulation) and can be used in prophylaxis, therapy, follow-up or aftercare of patients who suffer from clinical symptoms and/or known exposure to COVID-19.

### Combination Treatment

In various embodiments, the active ingredient may be administered alone or in combination with one or more additional therapeutic agents. A synergistic or augmented effect may be achieved by using more than one compound in the pharmaceutical composition. The active ingredients can be used either simultaneously or sequentially.

In one embodiment, the TLR 7/8 inhibitor is administered in combination with one or more additional therapeutic agents. In one aspect of this embodiment, the one or more additional therapeutic agents is selected from anti-inflammatories, antibiotics, anti-coagulants, antiparasitic agent, antiplatelet agents and dual antiplatelet therapy, angiotensin converting enzyme (ACE) inhibitors, angiotensin II receptor blockers, beta-blockers, statins and other combination cholesterol lowering agents, specific cytokine inhibitors, complement inhibitors, anti-VEGF treatments, JAK inhibitors, immunomodulators, anti-inflammasone therapies, sphingosine-1 phosphate receptors binders, N-methyl-d-aspartate (NDMA) receptor glutamate receptor antagonists, corticosteroids, Granulocyte-macrophage colony-stimulating factor (GM-CSF), anti-GM-CSF, interferons, angiotensin receptor-neprilysin inhibitors, calcium channel blockers, vasodilators, diuretics, muscle relaxants, and antiviral medications.

In one embodiment, the TLR 7/8 inhibitor is administered in combination with an antiviral agent. In one aspect of this embodiment, the antiviral agent is remdesivir. In another aspect of this embodiment, the antiviral agent is lopinavir-ritonavir, alone or in combination with ribavirin and interferon-beta.

In one embodiment, the TLR 7/8 inhibitor is administrated in combination with a broad-spectrum antibiotic.

In one embodiment, the TLR 7/8 inhibitor is administered in combination with chloroquine or hydroxychloroquine. In one aspect of this embodiment, the TLR 7/8 inhibitor is further combined with azithromycin.

In one embodiment, the TLR 7/8 inhibitor is administered in combination with interferon-1-beta (Rebif^{®}).

In one embodiment, the TLR 7/8 inhibitor is administered in combination with dexamethasone.

In one embodiment, the TLR 7/8 inhibitor is administered in combination with one or more additional therapeutic agents selected from hydroxychloroquine, chloroquine, ivermectin, tranexamic acid, nafamostat, virazole, ribavirin, lopinavir/ritonavir, favipiravir, arbidol, leronlimab, interferon beta-1a, interferon beta-1b, beta-interferon, azithromycin, nitrazoxamide, lovastatin, clazakizumab, adalimumab, etanercept, golimumab, infliximab, sarilumab, tocilizumab, anakinra, emapalumab, pirfenidone, belimumab, rituximab, ocrelizumab, anifrolumab, ravulizumab-cwvz, eculizumab, bevacizumab, heparin, enoxaparin, apremilast, coumadin, baricitinib, ruxolitinib, dapagliflozin, methotrexate, leflunomide, azathioprine, sulfasalazine, mycophenolate mofetil, colchicine, fingolimod, ifenprodil, prednisone, cortisol, dexamethasone, methylprednisolone, melatonin, otilimab, ATR-002, APN-01, camostat mesylate, brilacidin, IFX-1, PAX-1-001, BXT-25, NP-120, intravenous immunoglobulin (IVIG), and solnatide.

In one embodiment, the TLR 7/8 inhibitor is administered in combination with one or more anti-inflammatory agent. In one aspect of this embodiment, the anti-inflammatory agent is selected from corticosteroids, steroids, COX-2 inhibitors, and non-steroidal anti-inflammatory drugs (NSAID). In one aspect of this embodiment, the anti-inflammatory agent is diclofenac, etodolac, fenoprofen, flurbirprofen, ibuprofen, indomethacin, meclofenamate, mefenamic acid, meloxicam, nabumetone, naproxen, oxaprozin, piroxicam, sulindac, tolmetin, celecoxib, prednisone, hydrocortisone, fludocortisone, bethamethasone, prednisolone, triamcinolone, methylprednisone, dexamethasone, fluticasone, and budesonide (alone or in combination with formoterol, salmeterol, or vilanterol).

In one embodiment, the TLR 7/8 inhibitor is administered in combination with one or more immune modulators. In one aspect of this embodiment the immune modulator is a calcineurin inhibitor, antimetabolite, or alkylating agent. In another aspect of this embodiment, the immune modulator is selected from azathioprine, mycophenolate mofetil, methotrexate, dapson, cyclosporine, cyclophosphamide, and the like.

In one embodiment, the TLR 7/8 inhibitor is administered in combination with one or more antibiotics. In one aspect of this embodiment, the antibiotic is a broad-spectrum antibiotic. In another aspect of this embodiment, the antibiotic is a pencillin, anti-straphylococcal penicillin, cephalosporin, aminopenicillin (commonly administered with a betalactamase inhibitor), monobactam, quinoline, aminoglycoside, lincosamide, macrolide, tetracycline, glycopeptide, antimetabolite or nitroimidazole. In a further aspect of this embodiment, the antibiotic is selected from penicillin G, oxacillin, amoxicillin, cefazolin, cephalexin, cephotetan, cefoxitin, ceftriazone, augmentin, amoxicillin, ampicillin (plus sulbactam), piperacillin (plus tazobactam), ertapenem, ciprofloxacin, imipenem, meropenem, levofloxacin, moxifloxacin, amikacin, clindamycin, azithromycin, doxycycline, vancomycin, Bactrim, and metronidazole.

In one embodiment, the TLR 7/8 inhibitor is administered in combination with one or more anti-coagulants. In one aspect of this embodiment, the anti-coagulant is selected from apixaban, dabigatran, edoxaban, heparin, rivaroxaban, and warfarin.

In one embodiment, the TLR 7/8 inhibitor is administered in combination with one or more antiplatlet agents and/or dual antiplatelet therapy. In one aspect of this embodiment, the antiplatelet agent and/or dual antiplatelet therapy is selected from aspirin, clopidogrel, dipyridamole, prasugrel, and ticagrelor.

In one embodiment, the TLR 7/8 inhibitor is administered in combination with one or more ACE inhibitors. In one aspect of this embodiment, the ACE inhibitor is selected from benazepril, captopril, enalapril, fosinopril, lisinopril, moexipril, perindopril, quinapril, ramipril and trandoliapril.

In one embodiment, the TLR 7/8 inhibitor is administered in combination with one or more angiotensin II receptor blockers. In one aspect of this embodiment, the angiotensin II receptor blocker is selected from azilsartan, candesartan, eprosartan, irbesartan, losartan, Olmesartan, telmisartan, and valsartan.

In one embodiment, the TLR 7/8 inhibitor is administered in combination with one or more beta-blockers. In one aspect of this embodiment, the beta-blocker is selected from acebutolol, atenolol, betaxolol, bisoprolol/hydrochlorothiazide, bisoprolol, metoprolol, nadolol, propranolol, and sotalol.

In another embodiment, the TLR 7/8 inhibitor is administered in combination with one or more alpha and beta-blocker. In one aspect of this embodiment, the alpha and beta-blocker is carvedilol or labetalol hydrochloride.

In one embodiment, the TLR 7/8 inhibitor is administered in combination with one or more interferons.

In one embodiment, the TLR 7/8 inhibitor is administered in combination with one or more angiotensin receptor-neprilysin inhibitors. In one aspect of this embodiment, the angiotensin receptor-neprilysin inhibitor is is sacubitril/valsartan.

In one embodiment, the TLR 7/8 inhibitor is administered in combination with one or more calcium channel blockers. In one aspect of this embodiment, the calcium channel blocker is selected from amlodipine, diltiazem, felodipine, nifedipine, nimodipine, nisoldipine, and verapamil.

In one embodiment, the TLR 7/8 inhibitor is administered in combination with one or more vasodilators. In one aspect of this embodiment, the one or more vasodilator is selected from isosorbide dinitrate, isosorbide mononitrate, nitroglycerin, and minoxidil.

In one embodiment, the TLR 7/8 inhibitor is administered in combination with one or more diuretics. In one aspect of this embodiment, the one or more diuretics is selected from acetazolamide, amiloride, bumetanide, chlorothiazide, chlorthalidone, furosemide, hydrochlorothiazide, indapamide, metalozone, spironolactone, and torsemide.

In one embodiment, the TLR 7/8 inhibitor is administered in combination with one or more muscle relaxants. In one aspect of this embodiment, the muscle relaxant is an antispasmodic or antispastic. In another aspect of this embodiment, the one or more muscle relaxants is selected from casisoprodol, chlorzoxazone, cyclobenzaprine, metaxalone, methocarbamol, orphenadrine, tizanidine, baclofen, dantrolene, and diazepam.

In one embodiment, the TLR 7/8 inhibitor is administered in combination with one or more antiviral medications. In one aspect of this embodiment, the antiviral medication is remdesivir.

In one embodiment, the TLR 7/8 inhibitor is administered in combination with one or more additional therapeutic agents selected from antiparasitic drugs (including, but not limited to, hydroxychloroquine, chloroquine, ivermectin), antivirals (including, but not limited to, tranexamic acid, nafamostat, virazole [ribavirin], lopinavir/ritonavir, favipiravir, leronlimab, interferon beta-1a, interferon beta-1b, beta-interferon), antibiotics with intracellular activities (including, but not limited to azithromycin, nitrazoxamide), statins and other combination cholesterol lowering and anti-inflammatory drugs (including, but not limited to, lovastatin), specific cytokine inhibitors (including, but not limited to, clazakizumab, adalimumab, etanercept, golimumab, infliximab, sarilumab, tocilizumab, anakinra, emapalumab, pirfenidone), complement inhibitors (including, but not limited to, ravulizumab-cwvz, eculizumab), anti-VEGF treatments (including, but not limited to, bevacizumab), anti-coagulants (including, but not limited to, heparin, enoxaparin, apremilast, coumadin), JAK inhibitors (including, but not limited to, baricitinib, ruxolitinib, dapagliflozin), anti-inflammasone therapies (including, but not limited to, colchicine), sphingosine-1 phosphate receptors binders (including, but not limited to, fingolimod), N-methyl-d-aspartate (NDMA) receptor glutamate receptor antagonists (including, but not limited to, ifenprodil), corticosteroids (including, but not limited to, prednisone, cortisol, dexamethasone, methylprednisolone), GM-CSF, anti-GM-CSF (otilimab), ATR-002, APN-01, camostat mesylate, arbidol, brilacidin, IFX-1, PAX-1-001, BXT-25, NP-120, intravenous immunoglobulin (IVIG), and solnatide.

In some embodiments, the combination of a TLR inhibitor with one or more additional therapeutic agents reduces the effective amount (including, but not limited to, dosage volume, dosage concentration, and/or total drug dose administered) of the TLR inhibitor and/or the one or more additional therapeutic agents administered to achieve the same result as compared to the effective amount administered when the TLR inhibitor or the additional therapeutic agent is administered alone. In some embodiments, the combination of a TLR inhibitor with the additional therapeutic agent reduces the total duration of treatment compared to administration of the additional therapeutic agent alone. In some embodiments, the combination of a TLR inhibitor with the additional therapeutic agent reduces the side effects associated with administration of the additional therapeutic agent alone. In some embodiments, the combination of an effective amount of the TLR inhibitor with the additional therapeutic agent is more efficacious compared to an effective amount of the TLR inhibitor or the additional therapeutic agent alone. In one embodiment, the combination of an effective amount of the TLR inhibitor with the one or more additional therapeutic agent results in one or more additional clinical benefits than administration of either agent alone.

As used herein, the terms "treatment," "treat," and "treating" refer to reversing, alleviating, delaying the onset of, or inhibiting the progress of a viral infection, or one or more symptoms thereof, as described herein. In some embodiments, treatment is administered after one or more symptoms have developed. In other embodiments, treatment is administered in the absence of symptoms. For example, treatment is administered to a susceptible individual prior to the onset of symptoms (e.g., in light of a known exposure to an infected person and/or in light of comorbidities which are predictors for severe disease, or other susceptibility factors).

### EXEMPLIFICATION

As depicted in the Examples below, in certain exemplary embodiments, compounds are prepared according to the following general procedures. It will be appreciated that, although the general methods depict the synthesis of certain compounds of the present invention, the following general methods, and other methods known to one of ordinary skill in the art, can be applied to all compounds and subclasses and species of each of these compounds, as described herein.

The symbols and conventions used in the following descriptions of processes, schemes, and examples are consistent with those used in the contemporary scientific literature, for example, the Journal of the American Chemical Society or the Journal of Biological Chemistry.

### Example 1: Synthesis of cis-5-(3-Amino-5-trifluoromethyl-piperidin-1-yl)-quinoline-8-carbonitrile (Compound 1)

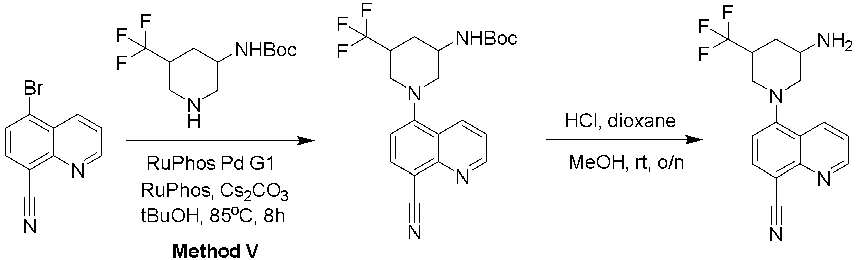

**Cis-[1-(8-cyano-quinolin-5-yl)-5-trifluoromethyl-piperidin-3-yl]-carbamic acid tert-butyl ester:** A mixture of 5-bromo-quinoline-8-carbonitrile (500 mg; 2.15 mmol), cis-3-(boc-amino)-5-(trifluormethyl)piperidine (691 mg; 2.57mmol), chloro(2-dicyclohexylphosphino-2',6'-di-i-propoxy-1,1'-biphenyl)[2-(2-aminoethylphenyl)]palladium(II), methyl-t-butylether adduct (88 mg; 0.11 mmol), 2-dicyclohexylphosphino-2',6'-di-i-propoxy-1,1'-biphenyl (50 mg; 0.11 mmol) and cesium carbonate (1.4 g; 4.3 mmol) in anhydrous *tert-*butanol (15 mL) was microwaved at 85°C for 8h. The reaction mixture was concentrated under reduced pressure and purified by chromatography, eluting with hexanes and ethyl acetate to afford [1-(8-cyano-quinolin-5-yl)-5-trifluoromethyl-piperidin-3-yl]-carbamic acid tert-butyl ester (731 mg; 80%) as a light yellow solid. ¹H NMR (400 MHz, CDCl₃) δ 9.09 (dd, *J* = 4.2, 1.7 Hz, 1H), 8.44 (dd, *J* = 8.6, 1.7 Hz, 1H), 8.04 (d, *J =* 7.9 Hz, 1H), 7.57 (ddd, *J* = 8.3, 6.2, 4.2 Hz, 1H), 7.12 (d, *J* = 8.0 Hz, 1H), 4.50 (s, 1H), 4.06 (s, 1H), 3.75 (dd, *J =* 11.8, 4.6 Hz, 1H), 3.65 - 3.55 (m, 1H), 2.89 (t, *J =* 11.3 Hz, 1H), 2.79 (dtq, *J =* 15.3, 7.5, 3.8 Hz, 1H), 2.56 - 2.39 (m, 2H), 1.54 - 1.33 (m, 10H); MS: *m*/*z* = 421 [M+H]⁺.

**Cis-5-(3-Amino-5-trifluoromethyl-piperidin-1-yl)-quinoline-8-carbonitrile: To a** solution of [1-(8-cyano-quinolin-5-yl)-5-trifluoromethyl-piperidin-3-yl]-carbamic acid tert-butyl ester (720 mg; 1.71 mmol) in anhydrous methanol (17 mL) was added a solution of hydrochloric acid (12.8 mL; 51.4 mmol) 4M in dioxane and the orange solution was stirred at room temperature overnight. Ether (40 mL) was added to the reaction mixture and the orange solution was stirred at room temperature for 20 min. The orange suspension was filtered, the yellow solid was washed with ether and dried under vacuo to afford 5-(3-amino-5-trifluoromethyl-piperidin-1-yl)-quinoline-8-carbonitrile hydrochloride (571 mg; 94%) as a yellow solid.

**Compound 1:** ¹H NMR (400 MHz, D₂O) δ 8.94 (dd, *J =* 4.6, 1.7 Hz, 1H), 8.66 (dd, *J* = 8.6, 1.8 Hz, 1H), 8.17 (d, *J =* 8.1 Hz, 1H), 7.77 (dd, *J =* 8.8, 4.5 Hz, 1H), 7.34 (d, *J =* 8.2 Hz, 1H), 4.02 - 3.87 (m, 1H), 3.82 (dd, *J =* 11.5, 3.6 Hz, 1H), 3.71 (d, *J =* 11.8 Hz, 1H), 3.17 (td, *J =* 8.0, 3.9 Hz, 1H), 3.05 (td, *J =* 11.4, 8.8 Hz, 2H), 2.64 (d, *J =* 12.3 Hz, 1H), 1.81 (q, *J =* 12.2 Hz, 1H); MS: *m*/*z =* 321 [M+H]⁺.

### Example 2: Separation of compound 1 into isomer 1 (Compound 2) (5-((3S,5R)-3-Amino-5-trifluoromethyl-piperidin-1-yl)-quinoline-8-carbonitrile and isomer 2 (Compound 3) 5-((3R,5S)-3-Amino-5-trifluoromethyl-piperidin-1-yl)-quinoline-8-carbonitrile):

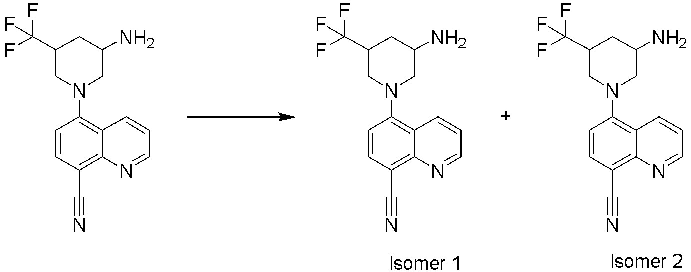

The title compounds were isolated via chiral SFC chromatography of compound 1. (Column: 2.1 x 25.0 cm Chiralpak AD-H from Chiral Technologies (West Chester, PA); CO2 Cosolvent (Solvent B): Methanol with 0.2% Ammonium Hydroxide; Isocratic Method: 20% Cosolvent at 80 g/min; System Pressure: 100 bar; Column Temperature: 25 °C).

**Compound 2:** ¹H NMR (400 MHz, Acetone-d6) δ 8.60 (dd, *J* = 4.2, 1.7 Hz, 1H), 8.06 (dd, *J* = 8.6, 1.7 Hz, 1H), 7.78 (d, *J =* 8.0 Hz, 1H), 7.24 (dd, *J =* 8.6, 4.2 Hz, 1H), 6.84 (d, *J =* 8.0 Hz, 1H), 3.07 (dt, *J* = 12.0, 2.3 Hz, 1H), 3.05 - 2.96 (m, 1H), 2.66 (tt, *J* = 11.2, 4.3 Hz, 1H), 2.57 (ddd, *J* = 15.5, 7.4, 3.4 Hz, 1H), 2.40 (t, *J* = 11.4 Hz, 1H), 2.04 - 1.97 (m, 1H), 1.78 - 1.67 (m, 1H), 1.29 (s, 2H), 0.80 (q, *J =* 12.1 Hz, 1H). MS: *m*/*z* = 321 [M+H]⁺.

**Compound 3:** ¹H NMR (400 MHz, Acetone-d6) δ 8.63 (dd, *J* = 4.2, 1.7 Hz, 1H), 8.12 (dd, *J* = 8.6, 1.7 Hz, 1H), 7.74 (d, *J =* 8.0 Hz, 1H), 7.12 (dd, *J* = 8.6, 4.2 Hz, 1H), 6.84 (d, *J* = 8.0 Hz, 1H), 3.09 (dt, *J* = 12.0, 2.3 Hz, 1H), 3.11 - 2.99 (m, 1H), 2.66 (tt, *J* = 11.2, 4.3 Hz, 1H), 2.58 (ddd, *J* = 15.5, 7.4, 3.4 Hz, 1H), 2.47 (t, *J* = 11.4 Hz, 1H), 2.07 - 1.79 (m, 1H), 1.75 - 1.67 (m, 1H), 1.23 (s, 2H), 0.84 (q, *J* = 12.1 Hz, 1H). MS: m/z = 321 [M+H]⁺.

### Example 3: Synthesis of (3R,5S)-1-(8-Methoxy-[1,7]naphthyridin-5-yl)-5-methyl-piperidin-3-ylamine (Compound 4)

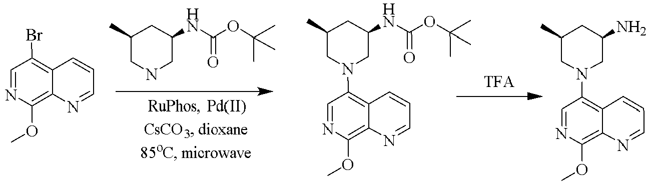

**[(3R,5S)-1-(8-Methoxy-[1,7]naphthyridin-5-yl)-5-methyl-piperidin-3-yl]-carbamic acid tert-butyl ester:** In a microwave vial, 5-Bromo-8-methoxy-[1,7]naphthyridine (0.58 g; 2.43 mmol; 1.0 eq.), ((3R,5S)-5-Methyl-piperidin-3-yl)-carbamic acid tert-butyl ester (0.62 g; 2.91 mmol; 1.20 eq.), chloro(2-dicyclohexylphosphino-2',6'-di-i-propoxy-1,1'-biphenyl)[2-(2-aminoethylphenyl)]palladium(ii), methyl-t-butylether adduct (99 mg; 0.12 mmol; 0.05 eq.), 2-dicyclohexylphosphino-2',6'-di-i-propoxy-1,1'-biphenyl (56 mg; 0.12 mmol; 0.05 eq.) and cesium carbonate (1.58 g; 4.85 mmol; 2.0 eq.) were dissolved in anhydrous Dioxane (11 ml). The reaction was placed under nitrogen and heated to 85°C in the microwave for eight hours. The reaction was purified on silica with an ethyl acetate/hexanes gradient to afford the title compound (578 mg; 1.55 mmol; 64.0%). MS: 373.5 [M+H]⁺.

**(3R,5S)-1-(8-Methoxy-[1,7]naphthyridin-5-yl)-5-methyl-piperidin-3-ylamine:** [(3R,5S)-1-(8-Methoxy-[1,7]naphthyridin-5-yl)-5-methyl-piperidin-3-yl]-carbamic acid tert-butyl ester (185.0 mg; 0.50 mmol; 1.0 eq.) was dissolved in dioxane (2 mL) in a reaction vial. Trifluoroacetic acid (4 mL; 2.48 mmol; 5.0 eq.) was added, and the reaction stirred for four hours. The mixture was purified via prep HPLC with an acetonitrile/water (0.1% NH₄OH modified) gradient to afford the title compound (114.0 mg; 0.42 mmol; 84.3%). MS: 273.4 [M+H]⁺. ¹H NMR (400 MHz, DMSO-d6) δ 8.94 (dd, *J* = 4.3, 2.1 Hz, 1H), 8.38 - 8.33 (m, 1H), 7.78 (dd, *J* = 8.8, 4.0 Hz, 1H), 7.73 (s, 1H), 4.02 (d, *J* = 1.8 Hz, 3H), 3.27 - 3.18 (m, 1H), 3.10 (d, *J* = 11.4 Hz, 2H), 2.98 (s, 2H), 2.28 (t, *J =* 10.8 Hz, 2H), 1.94 (s, 2H), 0.91 (d, *J =* 6.3 Hz, 3H), 0.80 (q, *J =* 12.1 Hz, 1H).

### Example 4: Pharmaceutical preparations

(A) Solution: A 100 mg powder in bottle solution was prepared from 100 mg of Compound 3, 100 g of sucrose, 18.2 g of citric acid monohydrate, and 5.3 g of sodium citrate in 930 mL of bidistilled water. The powder in bottle was agitated or shaken until all solutes dissolved. This solution is appropriate for oral administration by mouth or nasogastric tube.
(B) Tablets: 25 mg film coated tablets were produced containing:

| **Ingredient** | **Amount** |
|---|---|
| Compound 3 | 25.0 mg |
| Mannitol | 144.50 mg |
| Microcrystalline cellulose | 59.70 mg |
| Hyrpomellose | 9.55 mg |
| Croscarmellose sodium | 7.50 mg |
| Magnesium stearate | 3.75 mg |

Tablet ingredients were thoroughly mixed and compressed using standard techniques. After tablet formation, the tablet was coated with 5.0 mg of Opadry^{®} ZX321A220017 Yellow. These tablets are suitable for oral administration.

### Exmaple 5: TLR 7 and TLR 8 Stimulation

***Whole blood assay***: Blood was drawn from healthy donors into Vacutainer tubes with EDTA (BD Biosciences) and assay was performed within 2 hr. Four parts blood were diluted with one part PBS and 150 µl/well was dispensed into 96-well plates. Compound dilutions were added in triplicate and plates were incubated at 37°C 5% CO₂ for 30 min. For TLR7/8 testing, R848 (resiquimod) was added to 1µM, for TLR7, the TLR7 selective agonist shown below was added to 3 µM and for TLR8, the TLR8 selective agonist shown below was added to 0.5 µM. Plates were incubated overnight 37°C 5% CO₂ and cytokines were measured in the plasma by AlphaLISA.

Human whole blood was treated with multiple concentrations of Compound 3, Compound 4, and hydroxychloroquine before stimulation with TLR 7 and TLR 8-specific small molecule agonists. IL-6, TNF-alpha and IFN-alpha were measured in plasma 16h later.

The TLR 7 agonist used in the above experiment was: 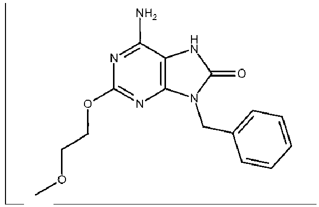 and the TLR 8 agonist used was:

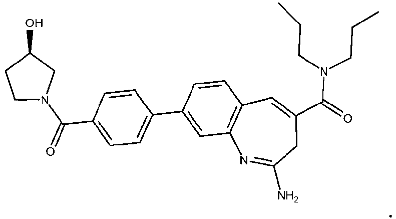

Compound 3 and 4 dose-dependently decreases IFNα and IL-6 production in mice challenged with the TLR-7 agonist, TLR-8 agonist, and TLR7/8 agonist R848 (Resiquimod, a known TLR 7/8 agonist). See Fig. 2A-D, and Fig. 4A-4E. Notably, IL-6 inhibition for Compound 3 is still 100% at low doses (0.1 mg/kg) where the corresponding IFNα inhibition at the same concentration is only partial, indicating Compound 3 has a more potent inhibition of the pro-inflammatory cytokines over the antiviral response.

### Example 6: In vivo inflammatory response

***BXSB-Yaa model*** -Male ***BXSB-Yaa*** mice were purchased from Jackson Labs and at 7-8 weeks of age treatment was started. Mice were dosed once per day via oral gavage with Compound 3 formulated in 0.1 M Na-citrate buffer pH 3. After 14 weeks of treatment, mice were euthanized via CO₂ asphyxiation and blood was collected via the vena cava. Plasma was collected from a portion of the blood and 100 µl of blood was preserved in animal blood protect tubes (Qiagen) for gene expression analysis. For monitoring of proteinuria in BXSB-*Yaa* mice, urine was collected by bladder massage in the morning on 2 consecutive days and the samples were pooled. The levels of albumin and creatinine in the urine were determined using the Advia 1800 clinical chemistry analyzer (Siemens). The urinary albumin-to-creatinine ratio (UACR) was calculated as the ratio of milligrams of albumin per gram of creatinine per deciliter of urine.

The experiments showed that Compound 3 reduces disease in mouse lupus models. ***BXSB-Yaa*** mice were dosed with Compound 3 via oral gavage. Survival was tracked over time (FIG 3A) and kidney disease was assayed by measuring proteinuria and plotted as a timecourse (FIG 3B) or the AUC for individual mice for the timecourse (FIG 3C). FIG 3D shows blood gene expression analysis which was performed on a panel of 17 IFN-regulated genes to calculate an IFN gene signature score relative to a healthy control mice. The proteinuria timecourse graph presents means + SEM and all other data is graphed as medians. Statistical significance was determined by Kruskal-Wallis for proteinuria (*p<0.05). and ANOVA for IFN scores (**p<0.01).

Compound 3 treatment of mice with TLR7-driven autoimmune disease (BXSB-*Yaa*) leads to a dose-dependent improvement in survival (Fig. 3A) and reduction in kidney disease as evidenced by lower proteinuria (Fig. 3B, 3C). These mice show a strong type I IFN response, which is inhibited by Compound 3 (Fig. 3D). Type I IFN inhibition is only seen at high doses, while immunopathology is improved at 10-100-fold lower doses consistent with preferential inhibition of TLR7/8-induced pro-inflammatory cytokines over antiviral type I IFN.

Results: Compound 3 is a potent, selective TLR7/8 inhibitor, that dose-dependently inhibits pro-inflammatory cytokines (e.g. IL6, TNF-alpha) which are induced by ssRNA viruses like SARS-Cov-2 over anti-viral cytokines (e.g. IFN-alpha), thereby potentially reducing immune-mediated pathologies over anti-viral immunity. This immunotherapeutic strategy is substantially more targeted relative to broader immunosuppressants (e.g. pan JAK inhibitors like Baricitinib, corticosteroids and hydroxychloroquine) in clinical trials that may increase the risk of secondary bacterial infection. This data shows that administration of Compound 3 after the initial viral response phase (Fig. 1) would likely prevent the second phase of antibody-dependent elaboration of cytokinemia, while keeping initial viral clearance uncompromised.

### Example 7: Clinical Trial Protocol

The purpose of this study is to evaluate the safety and efficacy of orally-administered Compound 3 in COVID-19 pneumonia patients who are hospitalized but not yet admitted to the intensive care unit. The study is a 2 dose level evaluation of Compound 3 taken for 14 days in a randomized, double-blind, placebo-controlled design. The study design and participant safety monitoring are based on Compound 3 data obtained from the first-in-human Phase I single ascending dose and 14 day multiple ascending dose healthy volunteer study, nonclinical evaluations of Compound 3, and clinical studies of other anti-inflammatory agents (e.g., tocilizumab) for COVID-19. Compound 3 demonstrates dose-proportional pharmacokinetics (PK), has a half-life of ~7 to 11 hours, and is mainly metabolized by aldehyde oxidase, not the common CYP450 enzymes.

Compound 3 is a small molecule, dual Toll-like receptor (TLR) 7 and TLR8 antagonist shown to specifically inhibit the activity of various TLR7/8 ligands such ssRNA, certain GU-rich microRNAs, and small molecule receptor agonists. TLR7 and TLR8 are expressed in the endosomes of cells with innate immune function, where activation by ssRNA viruses (e.g., SARS-CoV-2) stimulates secretion of type I interferons (IFNs) and proinflammatory cytokines (interleukin 6 [IL-6], tumor necrosis factor alpha [TNFα] and others), cellular maturation and activation of other host immune mechanisms (Li et al; and Chow et al).

Two dose levels of Compound 3, 100 mg twice daily and 50 mg twice daily, will be evaluated against placebo. The dose selection is guided by PK and pharmacodynamics data from the Phase I healthy volunteer study and by the doses found to be efficacious in preclinical lupus animal models. In the Phase I study, Compound 3 suppressed secretion of ex vivo-stimulated cytokines including IL-6, TNFα, and IFNα in an exposure-dependent manner. Based on these data, preliminary modeling and simulations projected 100 mg twice daily would suppress ex vivo-stimulated IL-6 production by 90% in 87% of healthy volunteers, and 50 mg twice daily would suppress it by 50% in 90% of healthy volunteers. As the magnitude of TLR7 and TLR8 inhibition required for suppression of the explosive cytokine production observed in some COVID-19 patients who progress to acute respiratory disease syndrome is unknown, and the Compound 3 safety profile has not been described in this patient population, so clinical and pharmacologic evaluation of the 50 mg twice daily dose in participants with progressive COVID-19 pneumonia is viewed as justified.

Given the available data for Compound 3 in humans, a 2-part, placebo controlled study is deemed necessary. The study will begin with a focused assessment of safety in a few participants (Part A) before expanding to a full Phase II clinical evaluation of the drug. Study participants should not be simultaneously enrolled in other COVID-19 studies and other immunomodulating drugs should not be used with Compound 3. The study participant's primary managing clinician may place the participant on the locally preferred antiviral therapy (agreed to at the site before study implementation) with permission of the local Clinical Investigator.

For this proof of principle Phase II study, assessing patients who are clinically deteriorating from their COVID-19 pneumonia, the time to peripheral capillary oxygen saturation (SpO₂) ≥ 94% sustained for at least 24 hours in room air will be the primary outcome measure. The study will also evaluate additional clinical parameters (e.g., those accepted for severe influenza) as secondary endpoints to better inform future drug evaluation in this population. Specifically, at the end of day 14 of administration, the study will look at 1) whether the patient has been discharged from the hospital with no limitations on activities, 2) whether the patient is discharged but has limitations of activities, 3) whether the patient remains hospitalized, but is not requiring supplemental oxygen therapy, 4) whether the patient is hospitalized and is requiring supplemental oxygen by mask or nasal prongs, 5) whether the patient is hospitalized and on non-invasive ventilation or high-flow oxygen with FiO₂ < 0.50, or 6) whether the patient is hospitalized and on mechanical ventilation or ECMO, or 7) whether the patient has died of their disease.

Inclusion criteria:
1. Are ≥ 18 to ≤ 65 years of age, at the time of signing the informed consent.
2. Test positive for SARS-CoV-2 based on locally acceptable guidelines.
3. Documentation of Chest Imaging consistent with COVID-19 pneumonia.
4. Not on mechanical ventilation (invasive or non-invasive).
5. Have an SpO₂ < 94% in room air AND PaO₂/FiO₂ ≥ 150 with a maximum FiO₂ 0.4.
6. Require hospitalization.
7. Have a body mass index within the range of ≥ 18.5 and ≤ 35.0 kg/m².

Administration of Compound 3 may be done as a monotherapy, or in combination with one or more additional therapeutic agent. The FDA has recently given emergency use authorization (EUA) for treatment of COVID-19 pneumonia to remdesivir. If the consulting physician recommends that Compound 3 is co-administered with remdesivir, then data on the effectiveness of the combination will be generated. It is likewise envisioned that Compound 3 may be administered in combination with lopinavir/ritonavir and interferon 1-beta. Furthermore, Compound 3 may be co-administered with patients getting transfusions of convalensent plasma, if the consulting physician recommends the combination.

Data collected from this study will assess what dose and administration frequency is appropriate for a safe and effective treatment for adults with COVID-19 pneumonia.

### Example 8: Antiviral testing of Compounds 3 and 4

Calu-3 cells were seeded on two 384 well plates. Plate 1 contained compounds plus virus SARS-CoV2/ZG/297-20 Passage 6 0.05 multiplicity of infection and Plate 2 contained compounds only. For each well, 15,000 Calu-3 cells were seeded in 50 µL/well in full growth medium (EMEM, 10% FCS, 1% Pen/strep). The cells were grown for 48 hours at 37°C and 5% CO₂. After this time, the medium in both plates was changed and fresh medium was added to each well.

On plate 1: 5 µL of each compound with respective concentrations were added to the specified wells in duplicates for 1 hour, and were infected afterwards with SARS-Cov-2 in an MOI of 0.05. The final volume of each well contained 5 µL compound, 5 µL virus (diluted and amount adjusted to 0.05 MOI), and 40 µL EMEM full medium for a total of 50 µL per well. The plate was monitored by Incucyte microscopy after virus addition at 2h intervals, for a total observation time of 120 hours.

Viability of cells determined with Cell Glo reagent (Promega); 50 µL reagent was added to each well, incubated at RT in dark for 10 min, then the luminescence was measured with the Biotek plate reader.

Figures 5 and 6 clearly show that treatment with the Calu-3 cells with Compounds 3 and 4, respectively, provides for cell confluence similar to that of unifected cells by the 60 hour time point.

### EXAMPLE 9 - In vitro effect of administration of Compound 3

Blood was drawn from healthy donors and PBMCs isolated using gradient centrifugation. PBMCs were dispensed into 96-well plates and M5049 dilutions were added in triplicate to selected wells. Plates were incubated at 37°C and 5% CO₂ for 30 minutes. Then, RNA oligonucleotides were added to the cells at a concentration of 26 µg/ml for stimulation. The treated PBMCs were incubated overnight at 37°C and 5% CO₂, and IFNa, TNFa and IL-6 in the supernatant were measured the next day by AlphaLISA. Results are shown in Figures 7A-7C; M5049 (compound 3) significantly suppresses the production of IFNa, TNFa and IL-6 in the treated cells.
Sequences of RNA ligands used for stimulation are as follows: Let-7a, UGAGGUAGUAGGUUG UAUAGUU; Let-7b, UGAGGUAGUAGGUUGUGUGGUU; Let-7c, UGAGGUAGUAGGUUG UAUGGUU; Let-7e, UGAGGUAGGAGGUUGUAUAGUU; Let-7f, UGAGGUAGUAGAUUG UAUAGUU; miR-122, UGGAGUGUGACAAUGGUGUUUG; miR-223, CGUGUAUUUGAC AAGCUGAGUU; miR-21, UAGCUUAUCAGACUGAUGUUGA; miR-574, UGAGUGUGU GUGUGUGAGUGUGU.

### EXAMPLE 10 - In vivo effect of administration of Compound 3

Healthy female C57BL/6 mice were dosed with vehicle (0.1 M Na citrate pH 3.0) or increasing doses (0.1, 1, 10mg/kg) of M5049 p.o. After 30 min, mice were dosed with a single i.v. injection of 2mg/kg miR-122 (Sigma) complexed with Invivofectamine (Life Technologies). At 4 hours after miRNA dosing, mice were euthanized and blood was collected for cytokine analysis in plasma and lungs were collected for gene expression analysis. IFN-α and IL-6 were measured in the plasma samples by Alphalisa (Perkin Elmer). Lungs were homogenized using the OctoMacs and M tubes (Qiagen) in RLT buffer and RNA was extracted using the RNEasy mini kit (Qiagen). Gene expression analysis was performed using a custom NanoString panel. Gene signature scores were determined by calculating the Log2 fold change versus vehicle alone and then using the median Log2 fold change for all genes on the signature as the signature score for each mouse. Genes used for the IFN gene signature were: OAS1, OAS2, OAS3, OASL, BST2, CMPK2, GBP5, HERC6, IFI44, IFIT1, IFIT2, IFIT3, IFIH1, CXCL10, ISG15, MX1, MX2, STAT1, TNFSF10, USP18, RSAD2, and IRF7. Genes used for the NF-kB gene signature were: IL1RN, TNFAIP3, CSF1, IRF1, IL1B, IL6, NFKBIA, PTGS2, TAP1, and TNF. Mice treated with the liposomal formulation of miR-122 showed a significant increase in plasma levels of IFNa and IL-6 (paired student's t test), which was significantly reduced by M5049 in a dose-dependent manner (P<0.0074 for all concentrations for IFNa and P= 0.0237 for IL-6 at 1mg/g M5049 and P=0.0147 for IL-6 at 10mg/kg M5049 determined by One-way Anova). See Figures 8A and 8B. Administration of miR-122 lead to an increase of both, the IFN and NFkB gene signature scores indicating local lung inflammation. M5049 significantly (P<0.01, One-way ANOVA) reduced both gene signature scores at 1 and 10mg/kg indicating that M5049 distributes well to the lung and potently reduces lung inflammation. See Figures 9A and 9B.

While a number of embodiments of this invention are described herein, it is apparent that the basic examples may be altered to provide other embodiments that utilize the compounds and methods of this invention. Therefore, it will be appreciated that the scope of this invention is to be defined by the appended claims rather than by the specific embodiments that have been represented by way of example.

Aspects and features of the patent disclosure are set out in the following numbered items, which contain the subject matter of the claims of the parent application as originally filed
1. A method of treating a coronavirus infection in a subject in need thereof, comprising administering an effective amount of a TLR 7/8 inhibitor, or a pharmaceutically acceptable salt thereof, to the subject.
2. The method of item 1, wherein the coronavirus causes a SARS or MERS infection.
3. The method of item 1 or 2, wherein the coronavirus causes a SARS-CoV1 or SARS-CoV-2 or MERS-CoV infection.
4. The method of any one of items 1-3, wherein the coronavirus is SARS-CoV-2.
5. The method of any one of items 1-4, wherein the TLR 7/8 inhibitor is selected from the group consisting of: 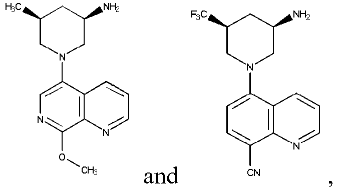 or a pharmaceutically acceptable salt thereof.
6. The method of item 1, wherein the TLR 7/8 inhibitor is: 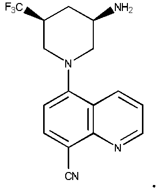
7. The method of any one of items 4-6, wherein the subject is suffering from a hyperinflammatory host immune response to a SARS-CoV-2 infection.
8. The method of any one of the preceding items, wherein the subject has COVID-19 pneumonia.
9. The method of any one of the preceding items wherein the subject has a moderate to severe COVID-19 which requires medical intervention.
10. The method of any one of items 7-9, wherein the hyperinflammatory host immune response is associated with one or more clinical indications selected from 1) reduced levels of lymphocytes, especially natural killer (NK) cells in peripheral blood; 2) high levels of inflammatory parameters (eg, C reactive protein [CRP], ferritin, d-dimer), and pro-inflammatory cytokines (eg, IL-6, TNFα, IL-8, and/or IL-1β); 3) a deteriorating immune system demonstrated by lymphocytopenia and/or atrophy of the spleen and lymph nodes, along with reduced lymphocytes in lymphoid organs; 4) dysfunction of the lung physiology represented by lung lesions infiltrated with monocytes, macrophages, and/or neutrophils, but minimal lymphocytes infiltration resulting in decreased oxygenation of the blood; 5) acute respiratory distress syndrome (ARDS); 6) vasculitis, 7) encephalitis, Guillain-Barre syndrome, and other neurologic disorders, 8) kidney dysfunction and kidney failure, and 9) hypercoagulability such as arterial thromboses, and 10) or any combination of above resulting in end-organ damage and death.
11. The method of any one of the preceding items, wherein the subject is an adult patient.
12. The method of any one of items 1-10, wherein the subject is a pediatric patient.
13. The method of items 12, wherein the pediatric patient is suffering from vasculitis, including Kawasaki disease and Kawasaki-like disease.
14. The method of any one of the preceding items, wherein the TLR 7/8 inhibitor is administered once or twice a day.
15. The method of any one of the preceding items, wherein the total amount of TLR 7/8 inhibitor administered is between about 50 mg and about 300 mg per day.
16. The method of any one of the preceding items, wherein 50 mg of the TLR 7/8 inhibitor is administered twice a day.
17. The method of any one of items 1-15, wherein 100 mg of the TLR 7/8 inhibitor is administered twice a day.
18. The method of any one of the preceding items, wherein the TLR 7/8 inhibitor is administered for about 7 days to about 21 days.
19. The method of items 18, wherein the TLR 7/8 inhibitor is administered for about 14 days.
20. The method of any one of items 1-15, wherein 100 mg of the TLR inhibitor is administered twice a day to the subject in need thereof for 14 days.
21. The method of any one of the preceding items, wherein the TLR 7/8 inhibitor is administered orally.
22. The method of any one of the preceding items, wherein the subject is being treated inpatient in a hospital setting.
23. The method of any one of items 1-21, wherein the subject is undergoing outpatient treatment for their SARS-CoV-2 infection.
24. The method of item 23, wherein the subject is undergoing outpatient treatment following discharge from inpatient treatment in a hospital setting.
25. The method of any one of the preceding items , wherein administration results in one or more clinical benefit for the subject.
26. The method of item 25, wherein the one or more clinical benefit is selected from the group comprising: reduction of duration of a hospital stay, reduction of the duration of time in the Intensive Care Unit (ICU), reduction in the likelihood of the subject being admitted to an ICU, reduction in the rate of mortality, reduction in the likelihood of kidney failure requiring dialysis, reduction in the likelihood of being put on non-invasive or invasive mechanical ventilation, reduction of the time to recovery, reduction in the likelihood supplemental oxygen will be needed, improvement or normalization in the peripheral capillary oxygen saturation (SpO₂ levels) without mechanical intervention, reduction of severity of the pneumonia as determined by chest imaging (eg, CT or chest X ray), reduction in the cytokine production, reduction of the severity of acute respiratory distress syndrome (ARDS), reduction in the likelihood of developing ARDS, clinical resolution of the COVID-19 pneumonia, improvement of the PaO₂/FiO₂ ratio, and reduction of the inflammatory response in the subject.
27. The method of item 26, wherein the reduction of the inflammatory response of the subject results in the reduction of proinflammatory cytokine release driven by NFκB IL-1b, IL-6, IL-8, IL-12, IL-18, IL-23, or IL-27, alone or in combination with inhibition of cytokine release driven by IRF3/7, such as type I IFNs, including IFN α and/or IFN β.
28. The method of item 26, wherein the one or more clinical benefits includes the improvement or normalization in the peripheral capillary oxygen saturation (SpO₂ levels) in the subject without mechanical ventilation or extracorporeal membrane oxygenation.
29. The method of item 26, wherein the one or more clinical benefits is reduction in the likelihood of being hospitalized, reduction in the likelihood of ICU admission, reduction in the likelihood being intubated (invasive mechanical ventilation), reduction in the likelihood supplemental oxygen will be needed, reduction in the length of hospital stay, reduction in the likelihood of mortality, reduction in likelihood of relapse, including the likelihood of rehospitalization.
30. The method of any one of the preceding items , further comprising administration of one or more additional therapeutic agent.
31. The method of item 30, wherein the one or more additional therapeutic agents is selected from anti-inflammatories, antibiotics, anti-coagulants, antiparasitic agent, antiplatelet agents and dual antiplatelet therapy, angiotensin converting enzyme (ACE) inhibitors, angiotensin II receptor blockers, beta-blockers, statins and other combination cholesterol lowering agents, specific cytokine inhibitors, complement inhibitors, anti-VEGF treatments, JAK inhibitors, immunomodulators, anti-inflammasone therapies, sphingosine-1 phosphate receptors binders, N-methyl-d-aspartate (NDMA) receptor glutamate receptor antagonists, corticosteroids, Granulocyte-macrophage colony-stimulating factor (GM-CSF), anti-GM-CSF, interferons, angiotensin receptor-neprilysin inhibitors, calcium channel blockers, vasodilators, diuretics, muscle relaxants, and antiviral medications.
32. The method of item 31, wherein the one or more additional therapeutic agents is an antiviral medication.
33. The method of item 32, wherein the one or more additional therapeutic agents is remdesivir.
34. The method of item 32, wherein the one or more additional therapeutic agents is lopinavir-ritonavir.
35. The method of item 34, wherein the one or more additional therapeutic agents further includes ribavirin and interferon-beta.
36. The method of item 31, wherein the one or more additional therapeutic agents is chloroquine or hydroxychloroquine.
37. The method of item 31, wherein the one or more additional therapeutic agents is dexamethasone.
38. The method of item 31, wherein the one or more additional therapeutic agents is interferon-beta-1a (Rebif^{®}).
39. The method of item 30, wherein the one or more additional therapeutic agent is selected from hydroxychloroquine, chloroquine, ivermectin, tranexamic acid, nafamostat, virazole, ribavirin, lopinavir/ritonavir, favipiravir, leronlimab, interferon beta-1a, interferon beta-1b, beta-interferon, azithromycin, nitrazoxamide, lovastatin, clazakizumab, adalimumab, etanercept, golimumab, infliximab, sarilumab, tocilizumab, anakinra, emapalumab, pirfenidone, belimumab, rituximab, ocrelizumab, anifrolumab, ravulizumab-cwvz, eculizumab, bevacizumab, heparin, enoxaparin, apremilast, coumadin, baricitinib, ruxolitinib, dapagliflozin, methotrexate, leflunomide, azathioprine, sulfasalazine, mycophenolate mofetil, colchicine, fingolimod, ifenprodil, prednisone, cortisol, dexamethasone, methylprednisolone, melatonin, otilimab, ATR-002, APN-01, arbidol, camostat mesylate, brilacidin, IFX-1, PAX-1-001, BXT-25, NP-120, intravenous immunoglobulin (IVIG), and solnatide.
40. The method of any one the preceding items, wherein the administration of the TLR 7/8 inhibitor selectively reduces the hyperinflammatory host immune response state while not interfering with the subject's natural interferon response to the viral infection.
41. The method of item 40, wherein the hyperinflammatory host immune response state is reduced before the subject suffers a severe cytokine storm.
42. The method of any one of items 1-6, wherein the administration of the TLR 7/8 inhibitor results in the reduction of the viral load in the subject.
43. The method of item 42, wherein administration of the TLR 7/8 inhibitor reduces the viral load by increasing the pH of the endosome, reducing the ability of the virus to enter cells, and/or interfering with the terminal glycosylation of cellular receptor ACE2.
44. The method of item 42 or 43, wherein the TLR 7/8 inhibitor is administered prior to COVID-19 pneumonia development.
45. The method of item 42 or 43, wherein the TLR 7/8 inhibitor is administered prior to the subject developing a cytokine storm.
46. The method of any one of items 42-45, wherein the subject has a mild to moderate SARS-CoV-2 infection.
47. The method of any one of items 42-46, wherein the subject has been previously vaccinated with a SARS-CoV-2 vaccine and develops vaccine-related exacerbation of infection, for example, an antibody-dependent enhancement or related antibody-mediated mechanisms of vaccine/antibody-related exacerbation.
48. The method of any one of items 44-46, wherein the subject is asymptomatic at the start of the administration regimen.
49. The method of item 48, wherein the subject has had known contact with a patient who has been diagnosed with a SARS-CoV-2 infection.
50. The method of any one of items 42-49, wherein the subject begins administration of the TLR 7/8 inhibitor prior to being formally diagnosed with SARS-CoV-2 infection.
51. The method of any one of items 42-50, wherein the administration of the TLR 7/8 inhibitor results in one or more clinical benefits.
52. The method of item 51, wherein the one or more clinical benefits is shortening the duration of infection, reduction of the likelihood of hospitalization, reduction in the likelihood of mortality, reduction in the likelihood of ICU admission, reduction in the likelihood being placed on mechanical ventilation, reduction in the likelihood supplemental oxygen will be needed, and/or reduction in the length of hospital stay.
53. The method of any one of items 42-52, wherein the subject is undergoing outpatient treatment.
54. The method of any one of items 42-53, further comprising administration of one or more additional therapeutic agent.
55. The method of item 54, wherein the one or more additional therapeutic agents is selected from anti-inflammatories, antibiotics, anti-coagulants, antiparasitic agent, antiplatelet agents and dual antiplatelet therapy, angiotensin converting enzyme (ACE) inhibitors, angiotensin II receptor blockers, beta-blockers, statins and other combination cholesterol lowering agents, specific cytokine inhibitors, complement inhibitors, anti-VEGF treatments, JAK inhibitors, immunomodulators, anti-inflammasone therapies, sphingosine-1 phosphate receptors binders, N-methyl-d-aspartate (NDMA) receptor glutamate receptor antagonists, corticosteroids, Granulocyte-macrophage colony-stimulating factor (GM-CSF), anti-GM-CSF, interferons, angiotensin receptor-neprilysin inhibitors, calcium channel blockers, vasodilators, diuretics, muscle relaxants, and antiviral medications.
56. The method of item 55, wherein the one or more additional therapeutic agents is an antiviral medication.
57. The method of item 56, wherein the one or more additional therapeutic agents is remdesivir.
58. The method of item 56, wherein the one or more additional therapeutic agents is lopinavir-ritonavir.
59. The method of item 58, wherein the one or more additional therapeutic agents further includes ribavirin and interferon-beta.
60. The method of item 54, wherein the one or more additional therapeutic agents is chloroquine or hydroxychloroquine.
61. The method of item 55, wherein the one or more additional therapeutic agents is dexamethasone.
62. The method of item 55, wherein the one or more additional therapeutic agents is interferon-1-beta (Rebif^{®}).
63. The method of item 54, wherein the one or more additional therapeutic agent is selected from hydroxychloroquine, chloroquine, ivermectin, tranexamic acid, nafamostat, virazole [ribavirin], lopinavir/ritonavir, favipiravir, leronlimab, interferon beta-1a, interferon beta-1b, beta-interferon, azithromycin, nitrazoxamide, lovastatin, clazakizumab, adalimumab, etanercept, golimumab, infliximab, sarilumab, tocilizumab, anakinra, emapalumab, pirfenidone, ravulizumab-cwvz, eculizumab, bevacizumab, heparin, enoxaparin, apremilast, coumadin, baricitinib, ruxolitinib, dapagliflozin, colchicine, fingolimod, ifenprodil, prednisone, cortisol, dexamethasone, methylprednisolone, GM-CSF, otilimab, ATR-002, APN-01, camostat mesylate, arbidol, brilacidin, IFX-1, PAX-1-001, BXT-25, NP-120, intravenous immunoglobulin (IVIG), and solnatide.
64. The method of any one of items 42-63, wherein the TLR 7/8 inhibitor is administered once or twice a day.
65. The method of item 64, wherein the total amount of TLR 7/8 inhibitor administered is between about 50 mg and about 300 mg per day.
66. The method of item 65, wherein 50 mg of the TLR 7/8 inhibitor is administered twice a day.
67. The method of item 65, wherein 100 mg of the TLR 7/8 inhibitor is administered twice a day.
68. The method of any one of items 64-67, wherein the TLR 7/8 inhibitor is administered for about 7 days to about 21 days.
69. The method of item 68, wherein the TLR 7/8 inhibitor is administered for about 14 days.
70. The method of item 67, wherein 100 mg of the TLR inhibitor is administered twice a day to the subject in need thereof for 14 days.
71. The method of any one of items 64-70, wherein the TLR 7/8 inhibitor is administered orally.

## Claims

1. A TLR 7/8 inhibitor for use in a method of treating a coronavirus infection in a subject in need thereof, comprising administering an effective amount of the TLR 7/8 inhibitor, or a pharmaceutically acceptable salt thereof, to the subject.

2. The TLR 7/8 inhibitor for use of claim 1, wherein the coronavirus causes a SARS or MERS infection.

3. The TLR 7/8 inhibitor for use of claim 1 or 2, wherein the coronavirus causes a SARS-CoV1 or SARS-CoV-2 or MERS-CoV infection; optionally wherein the coronavirus is SARS-CoV-2.

4. The TLR 7/8 inhibitor for use of any one of claims 1-3, wherein the TLR 7/8 inhibitor is selected from the group consisting of: 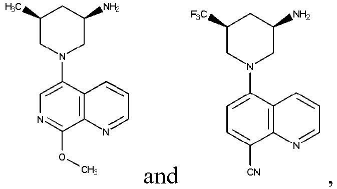 or a pharmaceutically acceptable salt thereof.

5. The TLR 7/8 inhibitor for use of claims 4, wherein the subject is suffering from a hyperinflammatory host immune response to a SARS-CoV-2 infection.

6. The TLR 7/8 inhibitor for use of any one of the preceding claims, wherein the subject has COVID-19 pneumonia.

7. The TLR 7/8 inhibitor for use of any one of the preceding claims wherein the subject has a moderate to severe COVID-19 which requires medical intervention.

8. The TLR 7/8 inhibitor for use of any one of the preceding claims, wherein the TLR 7/8 inhibitor is administered once or twice a day.

9. The TLR 7/8 inhibitor for use of any one of the preceding claims, wherein the total amount of TLR 7/8 inhibitor administered is between about 50 mg and about 300 mg per day.

10. The TLR 7/8 inhibitor for use of any one of the preceding claims, wherein 50 mg or 100 mg of the TLR 7/8 inhibitor is administered twice a day.

11. The TLR 7/8 inhibitor for use of any one of the preceding claims, wherein the TLR 7/8 inhibitor is administered for about 7 days to about 21 days; or wherein the TLR 7/8 inhibitor is administered for about 14 days.

12. The TLR 7/8 inhibitor for use of any one of the preceding claims, wherein the TLR 7/8 inhibitor is administered orally.

13. The method of any one of the preceding claims, further comprising administration of one or more additional therapeutic agent; optionally wherein the one or more additional therapeutic agents is selected from anti-inflammatories, antibiotics, anti-coagulants, antiparasitic agent, antiplatelet agents and dual antiplatelet therapy, angiotensin converting enzyme (ACE) inhibitors, angiotensin II receptor blockers, beta-blockers, statins and other combination cholesterol lowering agents, specific cytokine inhibitors, complement inhibitors, anti-VEGF treatments, JAK inhibitors, immunomodulators, anti-inflammasone therapies, sphingosine-1 phosphate receptors binders, N-methyl-d-aspartate (NDMA) receptor glutamate receptor antagonists, corticosteroids, Granulocyte-macrophage colony-stimulating factor (GM-CSF), anti-GM-CSF, interferons, angiotensin receptor-neprilysin inhibitors, calcium channel blockers, vasodilators, diuretics, muscle relaxants, and antiviral medications.

14. The method of claim 13, wherein the one or more additional therapeutic agents is an antiviral medication, optionally wherein the one or more additional therapeutic agents is remdesivir, or lopinavir-ritonavir alone or in combination with ribavirin and interferon-beta; or wherein the one or more additional therapeutic agents is chloroquine or hydroxychloroquine; or wherein the one or more additional therapeutic agents is dexamethasone; or wherein the one or more additional therapeutic agents is interferon-beta-1a (Rebif^{®}).

15. The method of claim 13, wherein the one or more additional therapeutic agent is selected from hydroxychloroquine, chloroquine, ivermectin, tranexamic acid, nafamostat, virazole, ribavirin, lopinavir/ritonavir, favipiravir, leronlimab, interferon beta-1a, interferon beta-1b, beta-interferon, azithromycin, nitrazoxamide, lovastatin, clazakizumab, adalimumab, etanercept, golimumab, infliximab, sarilumab, tocilizumab, anakinra, emapalumab, pirfenidone, belimumab, rituximab, ocrelizumab, anifrolumab, ravulizumab-cwvz, eculizumab, bevacizumab, heparin, enoxaparin, apremilast, coumadin, baricitinib, ruxolitinib, dapagliflozin, methotrexate, leflunomide, azathioprine, sulfasalazine, mycophenolate mofetil, colchicine, fingolimod, ifenprodil, prednisone, cortisol, dexamethasone, methylprednisolone, melatonin, otilimab, ATR-002, APN-01, arbidol, camostat mesylate, brilacidin, IFX-1, PAX-1-001, BXT-25, NP-120, intravenous immunoglobulin (IVIG), and solnatide.
